(19)

Europäisches Patentamt

European Patent Office

Office européen des brevets

(11) **EP 4 177 347 A1**

(12) **EUROPEAN PATENT APPLICATION**
published in accordance with Art. 153(4) EPC

(43) Date of publication:
**10.05.2023 Bulletin 2023/19**

(21) Application number: **21838233.1**

(22) Date of filing: **06.07.2021**

(51) International Patent Classification (IPC):
*C12N 15/34* (2006.01)      *C12N 15/861* (2006.01)
*A61K 48/00* (2006.01)      *A61P 35/00* (2006.01)

(52) Cooperative Patent Classification (CPC):
**A61K 39/215; A61K 48/00; A61P 31/14;
A61P 35/00; C07K 14/01; C07K 14/165;
C12N 15/64; C12N 15/861**

(86) International application number:
**PCT/CN2021/104641**

(87) International publication number:
**WO 2022/007774 (13.01.2022 Gazette 2022/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.07.2020   CN 202010642745
10.09.2020   CN 202010945335**

(71) Applicant: **Jiaxing Anyu Biotechnology Co., Ltd
Jiaxing, Zhejiang 314000 (CN)**

(72) Inventors:
• **CHEN, Ping**
  **Jiaxing, Zhejiang 314000 (CN)**
• **LI, Na**
  **Jiaxing, Zhejiang 314000 (CN)**

• **ZHANG, Tingting**
  **Jiaxing, Zhejiang 314000 (CN)**
• **ZHONG, Xintao**
  **Jiaxing, Zhejiang 314000 (CN)**
• **ZHU, Zhigang**
  **Jiaxing, Zhejiang 314000 (CN)**
• **LI, Nan**
  **Jiaxing, Zhejiang 314000 (CN)**

(74) Representative: **Longland, Emma Louise et al
Venner Shipley LLP
Byron House
Cambridge Business Park
Cowley Road
Cambridge CB4 0WZ (GB)**

Remarks:
The complete document including Reference
Table(s) and the Sequence Listing(s) can be
downloaded from the EPO website

(54) **NOVEL CHIMPANZEE ADENOVIRUS VECTOR, CONSTRUCTION METHOD THEREFOR, AND APPLICATION THEREOF**

(57)    The invention provides a novel chimpanzee adenovirus vector from newly discovered and isolated chimpanzee adenovirus with unique HVR sequences and a method of its construction and application thereof, with higher viral titer, and provides a method for determining its viral titer. The invention creatively constructs a chimpanzee adenovirus circular vector through a shuttle plasmid, and knocks out E1 and E3 to construct a replication-defective chimpanzee adenovirus vector. The chimpanzee adenovirus vector described in this article has no preexisting antibody in the population, and the knockout of E1 and E3 is safe. At the same time, it is significantly different from human adenovirus type 5 E1 in 293 cells, which can greatly avoid recovery mutation (RCA) and make it more safe. The novel coronavirus vaccine constructed by the invention has strong stability and does not cause mutations after multipepassages, and it can induce strong humoral immunity and cellular response in mouse models.

**(Cont. next page)**

**FIG.17**

## Description

CROSS-REFERENCE TO RELATED APPLICATIONS

The present application claims priority to a Chinese prior application No. 2020106427453 and filed on July 06, 2020, and a Chinese prior application No. 202010945335.6 and filed on July 06, 2020; the entire contents of the two patent applications are incorporated herein as a portion of the present invention.

## Technical field

The invention relates to the technical field of genetic engineering and the field of immunology, in particular to a novel chimpanzee adenovirus vector and a construction method and application thereof.

## Background technology

Adenovirus is a common virus causing upper respiratory tract and lung diseases, and the human immune system will have a strong immune response to adenovirus. Therefore, it is necessary to transform adenovirus into harmless vaccine vector. After decades of research and development, human adenovirus type 5 vaccine vector is widely used. Adenovirus is very popular as vaccine virus, usually, the adenovirus vector used in gene therapy is mainly human adenovirus type 5 (AdHu5), but neutralizing antibodies against AdHu5 are common in the population, and about one third of the population has become immune to it in adulthood. The immune system of these populations will destroy vaccines instead of producing antibodies against viruses carrying inserted genes. Moreover, the Hexon protein of AdHu5 binds to coagulation factor X (FX) in blood, which leads to the accumulation of adenovirus in liver and affects the tumor targeting of AdHu5. Therefore, the efficacy and targeting of adenovirus type 5 need to be further improved and perfected.

Chimpanzee adenovirus provides us with another feasible method. Because chimpanzee adenoviruses such as AdC7 and AdC23 are rarely prevalent in people, there are generally no corresponding neutralizing antibodies in people, and they will not be neutralized by antibodies against human adenovirus type 5 (AdHu5). At the same time, Hexon protein of chimpanzee adenovirus does not bind to FX. Therefore, chimpanzee adenovirus vector can be developed into an ideal adenovirus vector platform, which can be used to prepare higher quality vaccine reagents, such as Novel Coronavirus vaccine, and maintain inherent safety.

COVID-19, which began to break out in large numbers all over the world in 2019, caused serious diseases and caused huge losses. Coronavirus is a large virus family, which is known to cause colds and more serious diseases such as Middle East Respiratory Syndrome (MERS) and Severe Acute Respiratory Syndrome (SARS). Novel Coronavirus is a new strain of coronavirus that has never been found in humans before.

Novel Coronavirus expressed 29 proteins, of which 16 were related to its replication, and 4 were structural proteins, Spike protein (S protein), Nucleocapsid protein (N protein) and Envelope protein (E protein). S protein is the most important surface protein of coronavirus. S protein can bind to human ACE2 (host cell receptor angiotensin converting enzyme 2), and its affinity is much higher than that of severe acute respiratory syndrome coronavirus (SARS-CoV). S protein is the key to enter cells and the target of antibody drugs. N protein is abundant in coronavirus, which is a highly immunogenic protein and participates in genome replication and cell signaling pathway regulation.

The antibodies in novel coronavirus pneumonia patients will disappear within a few months, and so may the antibodies provided by vaccines, which means that people can't get vaccinated once and for all, and the antibodies will fade in a short time. The sooner or later of fading depends on the antibody level at the highest point, which determines how long the antibodies can stay in the body. Therefore, how to improve the antibody level of novel coronavirus pneumonia virus in patients is the primary problem to be considered in preparing Novel Coronavirus vaccine.

After testing, the serum of the population does not contain the pre-stored antibody of chimpanzee adenovirus, so the immune system will not destroy the vaccine, which is conducive to the effective expression of the target antigen and improve the level of antibody. Therefore, chimpanzee adenovirus is a good platform as a vaccine vector. The titer of chimpanzee adenovirus is generally low, and the process of constructing vaccine vector is complex and difficult to be industrialized. Therefore, it is urgent to find a chimpanzee adenovirus vector with higher titer and form a complete process platform, which can be used for the preparation of various vaccines such as Novel Coronavirus vaccine.

## Summary of the invention

To solve traditional problems, the invention provides a brand-new chimpanzee adenovirus with higher virus titer. The defective chimpanzee adenovirus vector prepared by the invention can be used for preparing various vaccines such as Novel Coronavirus vaccine. The chimpanzee adenovirus vaccine vector provided by the invention has no preexisting antibody in the population, and the E1 region of the novel chimpanzee adenovirus vector is very different with the E1 region of human adenovirus type 5 in 293 cells, which can greatly avoid recovery mutation; at the same time, the novel chimpanzee adenovirus vector has the characteristics of higher stability, and can induce strong humoral immunity and cellular response in mouse model.

One of the first aspects, the present invention provides a new chimpanzee adenovirus comprising a hyper variable region HVR, and the hyper variable region HVR comprises a nucleotide sequence represented by any one or more of sequence represent by SEQ ID NO. 9, SEQ ID NO. 10, SEQ ID NO. 11, SEQ ID NO. 12, SEQ ID NO. 13, or SEQ ID NO. 14 or a nucleotide sequence having more than 50% homology with the above sequences including SEQ ID NO. 9, SEQ ID NO. 10, SEQ ID NO. 11, SEQ ID NO. 12, SEQ ID NO. 13, or SEQ ID NO. 14.

Human adenoviruses belong to adenoviridae and mammalian adenoviruses. Up to now, 52 serotypes of human adenoviruses have been isolated, belonging to a ~ G7 subgenera. At present, virus isolation technology, antibody detection technology and molecular biology technology (such as polymerase chain reaction, restriction fragment length polymorphism analysis technology, in situ hybridization technology, real-time fluorescence quantitative PCR technology) are mainly used in the diagnosis and typing of adenovirus infection.

Chimpanzee adenovirus also has different serotypes; it's gene sequence includes the sequence of hypervariable region HVR1-9 and relatively conservative sequence. The relatively conservative sequence can be used to distinguish different adenoviruses (such as chimpanzee adenovirus or human adenovirus), while the hypervariable region sequence can be used to distinguish different serotypes of adenoviruses, that is to say, the sequence of hypervariable region determines the specific serotype of adenovirus.

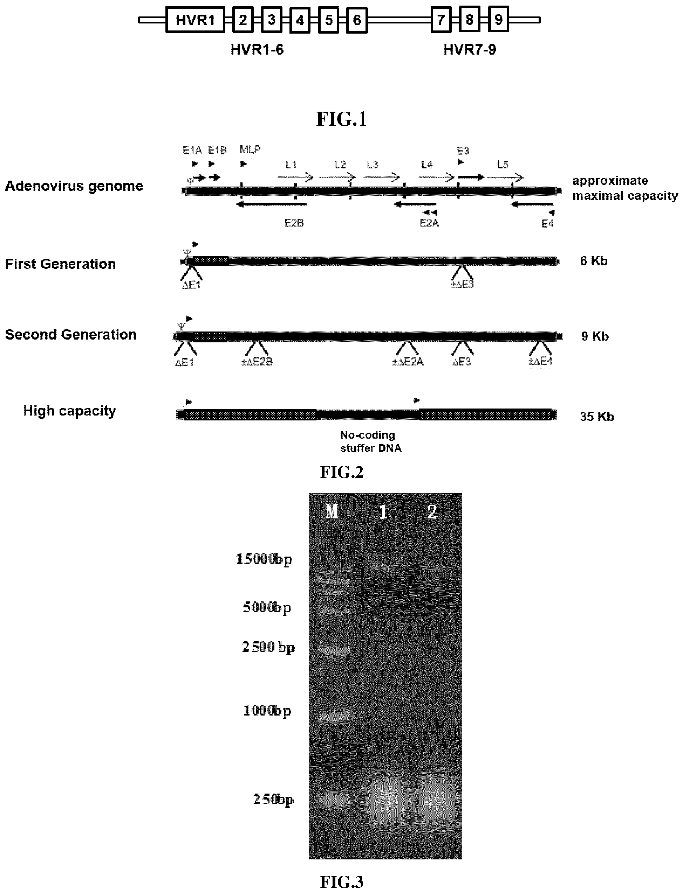

The sequence diagram of HVR1-9 is shown in Fig. 1. There are 9 regions in the hypervariable region. The different nucleic acid sequences of these hyper variable regions, thus the different expressed proteins or structures, determine the different serotypes of viruses. Generally, the hyper variable region of adenovirus has 9 regions, and the arrangement of these 9 regions is shown in Figure 1. The so-called "serotype" means that in microbiology, it can be used to identify different types of microorganisms of the same kind, which can usually be detected by serological methods, which is called serotype. For example, the serotype of Streptococcus is determined mainly by titration titer of O antigen and H antigen.

The gene sequence of the novel chimpanzee adenovirus provided by the invention is mainly based on the sequence of the hexon gene hypervariable region HVR1-6 of the new chimpanzee adenovirus. The sequence of each region in the sequence of the hexon gene hypervariable region HVR1-6 of the chimpanzee adenovirus provided by the present invention is completely or significant different from the sequence of the serotype HVR1-6 of the known chimpanzee adenovirus in the database (genebank). The HVR1-6 sequence of the hexon gene hypervariable region of the new chimpanzee adenovirus comprises six mutation sequences respectively located in each sequence of the HVR1-6 sequence: HVR1 is the sequence indicated by the sequence number SEQ ID NO. 9, HVR2 is the sequence indicated by the sequence number SEQ ID NO. 10, HVR3 is the sequence indicated by the sequence number SEQ ID NO. 11, HVR4 is the sequence indicated by the sequence number SEQ ID NO. 12, HVR5 is the sequence indicated by the sequence number SEQ ID NO. 13, and HVR6 is the nucleotide sequence indicated by the sequence number SEQ ID NO. 14. The sequence of the adenovirus of the present invention at the position of HVR7-9 in the hypervariable region is not mutated, as shown in the sequence SEQ ID NO. 15.

The person with ordinary skill in the present invention will appreciate that the sequences of these hypervariable regions, once published, may be modified, artificially synthesized, or other unrelated sequences added to these sequences. These sequences, which have been modified, artificially synthesized, or added to 3 or 5 segments of the sequences consolidated by the invention, Thus forming a new sequence, Although these new sequences are not 100% the same as those published in the present invention, However, the scope of the present invention can still cover sequences having homology with the sequences of the above hypervariable regions 1-6, These new sequences are also within the scope of protection of the present invention, where the new sequence homology may be a sequence homology of a region in a single hypervariable region, for example, a sequence having more than 50% homology with the sequence shown in sequence number Seq ID NO. 9, for example, 55%, 60%, 65%, 70%, 75%, 80%, 85%, 88%, 90%, 95%, or 99%. In addition, sequences such as those having 50% homology with the sequences one of the hypervariable regions 1-6 of the present invention, such as 55%, 60%, 65%, 70%, 75%, 80%, 85%, 88%, 90%, 95%, or 99%, are also possible, and these sequences are also one of the scope of the present invention. In addition, if the sequence of the present invention or the protein sequence encoded by the sequence having more than 50% homology with the sequence of the present invention can be encoded, it is also within the scope of protection of the present invention, that is, the protein or amino acid sequence encoded by these sequences is also within the scope of protection of the present invention.

Of course, these new sequences may include one or more of the nucleic acid sequences in the hypervariable regions of the invention, for example it includes one or more of the nucleic acid sequences shown in hypervariable regions 1-6, the latter comprising sequences having more than 50% homology with each of the nucleic acid sequences in the hypervariable regions 1-6 of the invention, for example 55%, 60%, 65%, 70%, 75%, 80%, 85%, 88%, 90%, 95%, or 99% homology.

Further, in some embodiments, the adenovirus comprises a region of the hypervariable region HVR7-9 having a nucleotide sequence as shown in the sequence as presented by SEQ ID NO. 15.

Further, the hypervariable region HVR has a nucleotide sequence as shown in the sequence SEQ ID NO. 7, or a nucleotide sequence having more than 50% homology with this sequence.

Further, the hypervariable region HVR has a nucleotide sequence as shown in the sequence SEQ ID NO. 7, or a nucleic acid sequence having 55%, 60%, 65%, 70%, 75%, 80%, 85%, 88%, 90%, 95%, or 99% homology therewith.

Further, the chimpanzee adenovirus also includes a conserved region.

On the second aspect, the invention provides a chimpanzee adenovirus, which has a preservation number of CCTCC NO: V202120 and a strain of sAd-AY01.

In some ways, the present invention provides an adenovirus comprising a nucleotide sequence as shown in the sequence table Seq ID NO.1, or a sequence having more than 50% homology with the sequence.

The invention provides a brand-new chimpanzee adenovirus. The whole genome of the chimpanzee adenovirus genome DNA is sequenced to determine the final virus genome sequence. The chimpanzee adenovirus was deposited in China Typical Culture Collection Center on April 25, 2021. It was classified as orangutan adenovirus name as sAd-AY01, the collection number was CCTCC NO: V202120, and the collection address was Wuhan University, China. The genome of this new chimpanzee adenovirus sAd-AY01 is 36687bp.

The brand-new chimpanzee adenovirus provided by the invention, It has a hypervariable region HVR sequence completely different from the existing inhibitory serotype. Compared with the known chimpanzee adenovirus partial serotype HVR1-6 sequence, monkey adenovirus partial serotype HVR1-6 sequence and human adenovirus partial serotype HVR1-6 sequence in gene bank, the homology is between 49% and 91%.

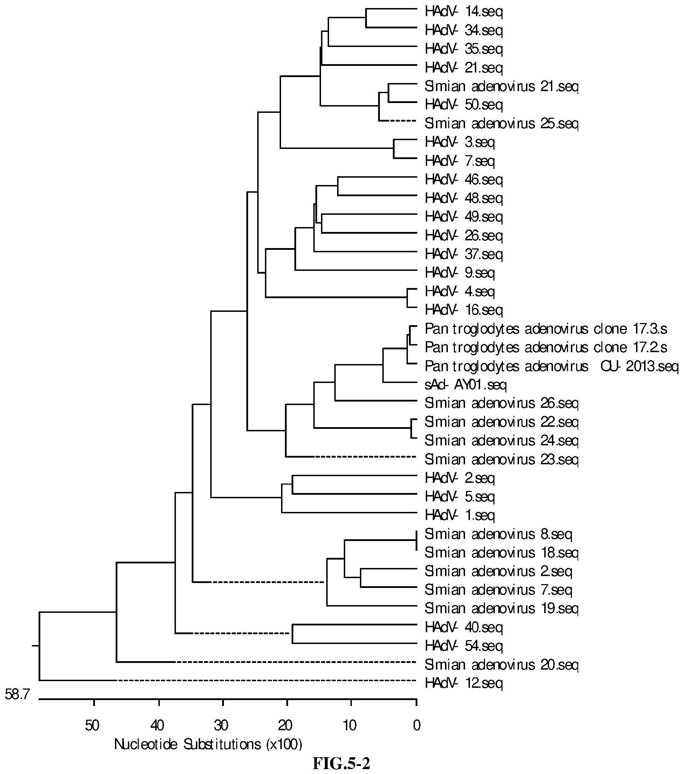

Based on the sequence of HVR1-6 in the hypervariable region of its hexon gene, it is compared with the known chimpanzee adenovirus partial serotype HVR1-6 sequence, monkey adenovirus partial serotype HVR1-6 sequence and human adenovirus partial serotype HVR1-6 sequence in genebank, the homology is between 49% and 91%. The phylogenetic tree was constructed by DNAstar software (Figure 5-2), and the homology and difference of sequences were analyzed by ClustalW calculation method. The adenovirus sequence with the largest homology was part of the gene of Pan troglodytes adenovirus clone 17.2 major capsid protein gene (GenBank: MF176134.1), and the largest homology was 91.63%. Finally, it was determined that the chimpanzee adenovirus was a new adenovirus that had not been discovered.

Since the discovery and successful isolation of adenoviruses in 1950s, more than 100 serotypes have been found one after another, among which there are at least 52 human adenoviruses, which are divided into seven subgroups: A, B, C, D, E, F and G. Human adenoviruses type 2 and type 5 commonly used in gene therapy belong to subgroup C in serological classification, and have 95% homology in DNA sequence. According to blood cell agglutination characteristics for chimpanzee adenovirus, twenty-two virus strain have been divided into three categories, each virus strain has an adenovirus complement immobilized antigen (Hillis WD, Goodman R (1969) Serological classification of chimpanzee adenoviruses by hemagglutination and hemagglutination inhibition.), the type-specific epitopes of adenovirus that can be recognized by neutralizing antibodies are mainly located on the hexon surface. The epitopes of hexon protein are plotted into two loops, L1 and L2, and are divided into seven hypervariable regions, HVR1-6 (L1) and HVR7 (L2). The hypervariable regions can lead to abnormal recombination. In most cases, HVR1-6 sequence sequencing can obviously distinguish serotypes. Molecular typing based on the hexon gene HVR1-7 and the discovery of new adenovirus variants have been widely used. (Casas A, Avellon M, Mosquera O, et a1. Molecular typing of human adenoviruses by PCR and sequencing of a partial region of the hexon gene. Lu X, Erdman DD. Molecular typing of human adenoviruses by PCR and sequencing of a partial region of the hexon gene. Arch Viro 1, 2006, 151: 1587-1602.).

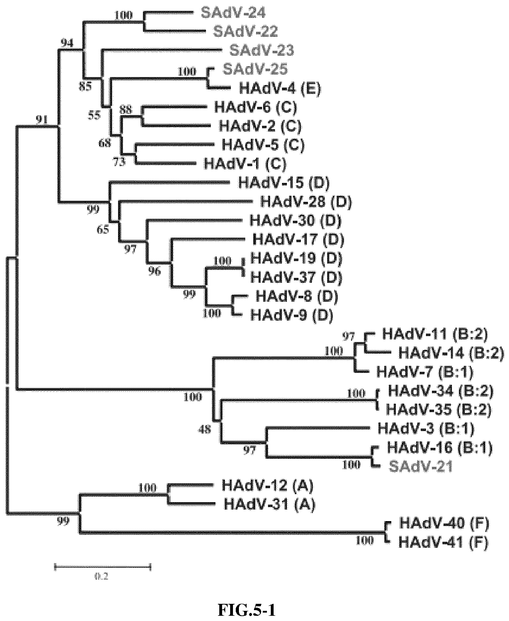

According to the reference of Complete nucleotide sequences and genome organization of four chimpanzee adenoviruses, chimpanzee adenovirus serotypes SAdV-22, SAdV-23, SAdV-24 (simian adenovirus 22, simian adenovirus 23, simian adenovirus 24) were classified into subtype E (as shown in Figure 5-1). The homology between simian adenovirus 22 and simian adenovirus 26 was 91.1%, the homology between simian adenovirus 24 and simian adenovirus 26 was 91.2%, simian adenovirus 22 and simian adenovirus 26 was 91.2%, and simian adenovirus 22 and Simian adenovirus 24 has a homology of 98.5%. According to the references, the sequences were aligned. Using DNAStar Megalign software and ClustalW algorithm, the reference sequences were synchronously aligned to construct a phylogenetic tree. Our serotype sAd-AY01 belongs to the same subtype as SAdV-22, SAdV-23 and SAdV-24 in references-subtype E, and is a new chimpanzee adenovirus.

The highest homology with the orangutan adenovirus sAd-AY01 provided by the invention is Pan troglodytes adenovirus clone 17.2 major capsid protein gene and Pan troglodytes adenovirus clone 17.2 major capsid protein gene. The two known chimpanzee adenoviruses are not preserved by any preservation institution at present, and only 2000bp sequences are disclosed.

The inventor has proved through a large number of studies that the novel chimpanzee adenovirus sAd-AY01 provided by the invention has very high virus titer, and the virus titer can reach $5 \times 10^8$ FFU/mL, which is far higher than the virus titer of the existing chimpanzee adenovirus and human adenovirus.

On the third aspect, the invention provides a new chimpanzee adenovirus vector psAd. The chimpanzee adenovirus vector psAd containing a HVR sequence in a hypervariable region is prepared by constructing a shuttle plasmid psAd-shuttle and carrying out homologous recombination with the chimpanzee adenovirus gene as described above after linearization.

In some embodiments, the plasmid contains a fluorescent labeled nucleic acid or a nucleic acid containing a fluorescent protein; the shuttle plasmid psAd-shuttle has a nucleotide sequence as shown in the sequence SEQ ID NO. 3, including replicon (pACYC), resistance gene (kanamycin), green fluorescent expression frame (RSV promoter, EGFP, BGHpolyA) nucleic acid.

In some embodiments, the cyclic plasmid psAd has a nucleotide sequence as shown in the sequence SEQ ID NO. 2, including a replicon (pACYC), a resistance gene (kanamycin), and a green fluorescent expression box (RSV promoter, EGFP, BGHpolyA) nucleic acid.

The chimpanzee adenovirus (sAd) genome is a linear DNA larger than 36kb in length, which is difficult to edit effectively. Therefore, it is necessary to connect the replication-related elements in bacteria, such as replicon (pACYC) and resistance gene (kanamycin), to the chimpanzee genome to form a circular plasmid.

The existing method of constructing cyclic plasmid is usually cosmid (phage), which needs to buy corresponding kits, and phage may also pollute the laboratory.

Shuttle plasmids are usually used as expression vectors to carry genes to be expressed into target plasmids. The invention creatively constructs a chimpanzee adenovirus vector ring plasmid by constructing a shuttle plasmid psAd-shuttle comprising a replicon (pACYC), a resistance gene (kanamycin), a green fluorescent expression frame (RSV promoter, EGFP, BGHpolyA), and then carries out homologous recombination with the chimpanzee adenovirus genome after linearization to form a chimpanzee ring plasmid, which can effectively replicate in bacteria; at the same time, in order to monitor the transfection efficiency of plasmid in cells, green fluorescent expression frames (RSV promoter, EGFP, BGHpolyA) were added, and the transfection efficiency of vector could be judged by the amount of fluorescence in TP0 generation after transfection into cells. It can be understood that these are not necessary for shuttle plasmids, such as resistance gene (kanamycin), green fluorescent expression box (RSV promoter, EGFP, BGHpolyA), and of course, green fluorescent expression box (RSV promoter, EGFP, BGHpolyA) is the least necessary.

The method for constructing the circular plasmid of the invention has the advantages that: 1, no need to purchase a reagent kit and avoid the possible contamination of the laboratory by using bacteriophage; 2. The transfection efficiency of TP0 can be tested by adding EGFP fluorescence sequence; 3. It is more flexible and can build the components it needs according to its own needs.

In yet another aspect, the present invention provides a replication-defective chimpanzee adenovirus vector based on the chimpanzee adenovirus described above, conserved region deletion gene.

It can be understood that on the basis of the circular plasmid prepared by the chimpanzee adenovirus sAd-AY01 provided by the invention, the replication-defective chimpanzee adenovirus vector can be prepared by deleting any gene.

In some ways, the deletion gene is associated with chimpanzee adenovirus replication, and the vector after deletion of the gene is a replication-deficient chimpanzee adenovirus vector.

In some ways, the deletion gene includes any one or more of E1, E2A, E2B, E3, E4. For example, deletion of one of E1, E2A, E2B, E3 and E4 regions, or deletion of any combination of multiple regions, such as E1 and E3; E1, E3 and E4; E1, E2A, E3 and E4; E1, E2A, E2B, E3, E4 and so on.

As shown in Fig. 2, the conserved region genes of the chimpanzee adenovirus vector provided by the present invention include E1, E2A, E2B, E3 and E4.

In some embodiments, replication-defective chimpanzee adenovirus vectors are missing genes in the E1 and E3 regions.

In some embodiments, replication-deficient chimpanzee adenovirus vectors can be constructed by deleting E2A, E2B and E4 regions in addition to E1 and E3 regions.

In some embodiments, the E1 and E3 genes are deleted and have a nucleotide sequence as shown in the sequence SEQ ID NO. 4.

Further, the invention provides a replication-defective chimpanzee adenovirus vector psAd △E1 △E3, which is based on the chimpanzee adenovirus described above , knocks out its E1 and E3 genes to obtain a chimpanzee adenovirus vector psAd △E1△ E3 deletion of E1 and E3 genes.

The replication-defective chimpanzee adenovirus vector comprises the sequence of the hypervariable region, the sequence of one region of the latter's six adjacent hypervariable regions or the region represented by a plurality of sequences.

The replication-deficient chimpanzee adenovirus vector psAd△ E1△ E3 has a nucleotide sequence as shown in the sequence SEQ ID NO. 4.

Wild-type chimpanzee adenovirus vector can replicate and amplify in ordinary cells. In application, this vector needs to lack its replication-related elements, and construct replication-deficient adenovirus vector, so that it can only replicate and package in specific complementary cell lines, but cannot replicate and package in ordinary cells. Therefore, the invention specifically knocks out the E1 and E3 genes of the chimpanzee adenovirus vector psAd, so that it can only be packaged in a specific cell line (such as the 293 cell line) and named as psAd△ E1△ E3. It will be understood that the adenovirus of the present invention also knocks out other sites, or adenoviruses with deletions of other sites, such as deletions of E2A, E2B, E4 sites, to obtain replication-defective adenovirus vectors.

In another aspect, the invention provides a method for constructing a replication-defective chimpanzee adenovirus vector, comprising the following steps:

(1) constructing the shuttle plasmid psAd, and carrying out homologous recombination with the chimpanzee adenovirus gene described in any one of the foregoing embodiments after linearization; Preferably, the shuttle plasmid comprises a fluorescent labeled cyclic plasmid psAd;
(2) knocking out the E1 gene of psAd to obtain the chimpanzee adenovirus vector psAd△E1 with deletion of E1 gene; optionally, the enzyme cutting site, such as the enzyme cutting site PsiI;, is introduced;
(3) the chimpanzee adenovirus vector psAd△E1△E3 was obtained by knocking out the E3 gene of psAd△E1.

Further, the shuttle plasmid psAd-shuttle in step 1) has a nucleotide sequence as shown in the sequence SEQ ID NO. 3, and the cyclic plasmid psAd has a nucleotide sequence as shown in the sequence SEQ ID NO. 2.

Further, in step 2), the E1 gene of psAd is digested by CRISPR/Cas9 enzyme, and an HRPT sequence containing a PsiI enzyme digestion site is inserted, and then purified and linked to obtain psAd△E1; the HRPT sequence has a nucleotide sequence as shown in the sequence SEQ ID NO. 5.

Further, step 3) through CRISPR/Cas9 enzyme digesting psAd△E1, the obtained product and the fusion fragment pVIII-U-exon are seamlessly cloned to obtain psAd△E1△E3; the fusion fragment pVIII-U-exon has a nucleotide sequence as shown in the sequence SEQ ID NO. 6.

Shuttle plasmids are widely used in the construction of adenovirus vectors, and it is necessary to find a single enzyme cleavage site. The invention creatively adopts CRISPR/cas9 to construct recombinant adenovirus vector, selects appropriate E1, E3, E4 and E2A knockout sites through comparison, selects CRISPR sites according to the positions of E1, E3, E4 and E2A sequences and the number of knockout gene bases, and designs the best gRNA, thereby completing the construction of recombinant adenovirus vector.

In yet another aspect, the present invention provides a vaccine comprising a target gene in a gene deletion region of the replication-defective chimpanzee adenovirus vector as described above.

It can be understood that the nucleic acid sequence inserted into the deletion region can be any nucleic acid sequence, such as infectious disease, tumor and the like, which can be obtained or expressed by the vector to prepare vaccine reagents.

In some embodiments, the gene deletion region includes any one or more of E1, E2A, E2B, E3, E4.

In some embodiments, the target gene is a virus, a bacterium, a tumor gene or a gene fragment.

In some embodiments, the target gene is the Novel Coronavirus S gene.

In some embodiments, the gene of interest is located in the E1 region of a defective chimpanzee adenovirus vector, which is shown to have a nucleotide sequence as shown in sequence SEQ ID NO. 4.

In some embodiments, the invention provides a novel coronavirus vaccine, which is obtained by constructing a recombinant chimpanzee adenovirus vector expressing novel Coronavirus S gene and packaging it with 293 cells; The S gene is expressed in the E1 region of the defective chimpanzee adenovirus vector as described above to form a recombinant adenovirus vector psAd-S.

In another aspect, the invention provides a construction method of a recombinant chimpanzee adenovirus vector expressing novel Coronavirus S gene, comprising the following steps:

(a) constructing the E1 region shuttle plasmid pSsAdEl of the chimpanzee adenovirus vector as described above;
(b) adding WPRE element to shuttle plasmid pSsAdEl to obtain pSsAdEl-WPRE element;
(c) construction of shuttle plasmid pSsAdE1-WPRE-S expressing complete S protein gene of COVID-19 in Novel Coronavirus;
(d) homologous recombination of shuttle plasmid pSsAdE1-WPRE-S and adenovirus vector plasmid psAd △E1△E3 formed recombinant adenovirus vector psAd-S.

Further, the WPRE element is shown to have a nucleotide sequence as shown in the sequence SEQ ID NO. 17.

In another aspect, the present invention provides a titer determination method of recombinant chimpanzee adenovirus, which detects the titer of chimpanzee adenovirus by detecting the change of HVR antigen epitope of hypermutant region by specific antibody prepared from hexon hypermutant region HVR gene fragment, said HVR gene having nucleotide sequence as shown in sequence SEQ ID NO. 7.

Further, the chimpanzee adenovirus is the chimpanzee adenovirus as described above, or the replication defective chimpanzee adenovirus vector, or the vaccine as described above, or any recombinant chimpanzee adenovirus vector, etc.

The novel chimpanzee adenovirus of the invention is quite different from the traditional human type 5 adenovirus and the traditional chimpanzee adenovirus. After detection, the titer detection of the chimpanzee adenovirus of the invention needs to prepare its unique antibody. The main coat proteins of the new chimpanzee adenovirus are hexon, penton and fiber, and the hypervariable region HVR of hexon is the key region to identify the serotype of adenovirus. Therefore, expressing HVR protein and preparing mouse serum can detect the titer of chimpanzee adenovirus.

The detection method of chimpanzee adenovirus titer provided by the invention can be used for detecting the titer of recombinant chimpanzee adenovirus psAd-S vaccine provided by the invention.

In another aspect, the invention provides a packaging method of a recombinant chimpanzee adenovirus vector, wherein the vector of psAd-S was digested with I-sceI enzyme, 293 cells were transfected with the linearized plasmid , and cell suspension was collected.

The beneficial effects of the invention are mainly embodied in:

(1) a new chimpanzee adenovirus is provided, and the virus titer can reach 5x10 ^ 8 FFU/ml;
(2) creatively constructed chimpanzee adenovirus ring vector through shuttle plasmid;
(3) the invention provides a brand-new construction method of CRISPR/cas9 of recombinant chimpanzee adenovirus vector, independently designs the best knockout site and gRNA, avoids the trouble of finding a single enzyme cutting site when shuttle plasmid knockout is used in vector construction in the past, and the construction method is simple, efficient and short in cycle;
(4) it provides a brand-new replication-deficient chimpanzee adenovirus vector, which has no pre-existing antibodies in the population,. At the same time, the E1 is very different from the E1 of human adenovirus type 5 in 293 cells, which can greatly avoid recovery mutation (RCA), the knockout of E1 and E3 make the chimpanzee adenovirus vector more safe;
(5) A new titer determination method of chimpanzee adenovirus is provided.
(6) After dozens of generations of passages, the constructed Novel Coronavirus vaccine is stability and have no mutation;
(7) The constructed Novel Coronavirus vaccine can induce strong humoral immunity and cellular response in mouse model.

**Illustrated drawings**

Fig. 1 is schematic diagram of HVR1-9 sequence of chimpanzee adenovirus gene hypervariable region.
Fig. 2 is schematic diagram of E1, E2A, E2B, E3 and E4 regions of chimpanzee adenovirus gene conserved region.
Fig. 3 is the results of the agarose gel electrophoresis confirmation of the genomic DNA sequence of the chimpanzee adenovirus in example 1, where M: 15000 bp Marker; 1, 2: Chimpanzee adenovirus genomic DNA.
Fig. 4is the results of comparison of the HVR1-6 sequence of the chimpanzee adenovirus sAd-AY01 in example 2 with the HVR1-6 sequence of some known serotypes.
Fig. 5-1 is schematic diagram of chimpanzee adenovirus serotyping in references.
Fig. 5-2 is schematic diagram of the process of constructing a phylogenetic tree analysis sequence of the HVR1-6 sequence of chimpanzee adenovirus sAd-AY01 and the known serotype HVR1-6 sequence in Example 2.
Fig. 6 is the electrophoresis results of enzyme digestion identification of the shuttle plasmid psAd-shuttle constructed in example 3, in which M: 15000bp Marker; 1: psAd-shuttle BamhI& EcoRI digestion
Fig. 7 is the plasmid map of the adenovirus genomic DNA cycling shuttle plasmid psAd-shuttle constructed in example 3.
Fig. 8 is the electrophoresis result of enzyme digestion identification of the chimpanzee adenovirus vector plasmid psAd constructed in example 3, in which M: 15000bp Marker; 1: psAd BamHI digestion; 2: psAd XhoI digestion.
Fig. 9 is the plasmid map of the chimpanzee adenovirus vector plasmid psAd constructed in example 3.
Fig. 10 is the agarose gel electrophoresis result of CRISPR/Cas9 "digested" psAd plasmid in example 4, where M: 15000bp Marker; 1: psAd "digested" E1 with cas9.
Fig. 11 is the agarose gel electrophoresis result of amplified fragment of HRPT sequence in example 4, wherein: M: 15000bp Marker; 1: HRPT fragment.
Fig. 12 is the result of identification electrophoresis after digestion of psAd△E1 construct in example 4.
Fig. 13 is the agarose gel electrophoresis result of CRISPR/Cas9 "digestion" psAd△E1 plasmid in example 4, where M: 15000bp Marker; 1, 2: psAd△E1 "digested" E3 with cas9.
Fig. 14 is the gel electrophoresis results of pVIII and U-exon fragments in example 4, where M: 15000bp Marker;

1: Partial fragment of PVIII; 2: U-exon partial fragment.

Fig. 15 is the gel electrophoresis result of pVIII-U-exon fragment amplified by fusion PCR in example 4.

Fig. 16 is the results of enzyme digestion identification electrophoresis of psAd△E1△E3 constructed in example 4, where M: 15000bp Marker; 1: psAd△E1△E3 BamHI digestion; 2: psAd△E1 △E3 XhoI digestion; The right picture shows the enzyme digestion simulation of psAd△E1△E3 plasmid.

Fig. 17 is the plasmid map of psAd△E1△E3 constructed in Example 4.

Fig. 18 is the gel electrophoresis results of PCR amplified left arm, puc & amp, right arm, CMV-MCS-SV40 sequences in example 5, wherein: M: 2000 bp Marker; 1 is the fragment left arm; 2 is the fragment puc& 3 is the fragment right arm; 4 is fragment CMV-MCS-SV40.

Fig. 19 is the results of enzyme digestion identification electrophoresis of the shuttle plasmid pSsAdEI constructed in example 5.

Fig. 20 is the enzyme digestion identification electrophoresis results of the pSsAdEI-WPRE element constructed in example 5, where M: 15000 bp Marker; 1, 2 were identified by pSsAdEI-WPRE NotI enzyme digestion; The pSsAdEI-WPRE component was successfully built.

Fig. 21 is the electrophoresis result of enzyme digestion identification of shuttle plasmid pSsAdE1-WPRE-S constructed in example 5, in which M: 2000 bp Marker; 1 and 2 are pSsAdE1-WPRE-S EcoRV & HindIII.

Fig. 22 is the results of enzyme digestion identification electrophoresis of the recombinant novel chimpanzee adenovirus vector psAd-S constructed in example 6, in which M: 15000 bp Marker; 1: psAd-S HindIII digestion; 2: psAd-S BamHI.

Fig. 23 is the e microscope photograph of cytopathic changes caused by TP0 to TP2 in example 7.

Fig. 24 is the e results of purification of the sAd-HVR protein obtained by NI column in example 8, where M: protein Maker; 1. supernatant of sAd-HVR bacteria liquid; 2: purified sAd-HVR protein; 3. Purified sAd-HVR protein diluent.

Fig. 25 is the Western Blot detection result of expression of psAd-S protein of recombinant novel chimpanzee adenovirus in example 11, wherein: M: 180kda maker; 1, 293 blank cell control; 2, 3: Samples of 293 cells infected with psAd-S virus.

Fig. 26 is the schematic diagram of cytopathic changes in example 12 using sAd-AY01 neutralizing antibody.

Fig. 27 is the schematic diagram of cytopathic changes in an experiment using an antibody neutralizing hAd5 in example 12.

Fig. 28 is the results of IgG antibody titer after intramuscular injection of psAd-S vaccine by ELISA in example 13.

Fig. 29 is the results of CD8 + T cell response induced by the vaccine psAd-S of example 13.

Fig. 30 is the results of CD4 + T cell response induced by the vaccine psAd-S of example 13.

Fig. 31 is the results of cellular immune response after intramuscular injection of vaccine psAd-S according to example 13.

Fig. 32 is A blank control immune response representative diagram of Example 13.

**Detail of Embodiments**

Preferred embodiments of the invention are described in further detail below in conjunction with the accompanying drawings and it should be noted that the embodiments described below are intended to facilitate understanding of the invention without any limiting effect thereon.

**EXAMPLE 1 Isolation of a novel chimpanzee virus strain**

Anal swabs from chimpanzees were collected, and chimpanzee adenovirus was cultured by HEK293 cells (purchased from ATCC, batch number 70009859).

First, the collected anal swab is melted on ice. Until room temperature, Put it in a 5ml cryopreservation tube, Add 3ml DMEM, Vortex 30s, 4 °C, 2000rpm, Centrifuge for 5min, The supernatant is filtered through a 0. 2 um pinhole filter membrane for about 200 um into a sterile 48-well plate for later use, Then inoculate HEK293 cells with 48 wells prepared in advance, and observe their pathological changes every day. If the cells are full in 48 hours, collect the cell suspension, freeze and thaw twice, and then freeze at-20 °C. If it is not full, collect the cell suspension on the 5th day, and continue to inoculate HEK293 cells until the pathological changes, and extract the virus genome for PCR amplification and identification. The Hirt Virial DNA Extract method was used to further expand the identified virus strains (the main steps were collecting 3.5 ml infected cells, freezing and thawing for 3 times, adding 20% SDS 87.5 ul, 20mg/ml Proteinase K 87.5 ul, incubating at 55 °C for 4 h; Add 1ml of 5 mol/L NaCl solution into the centrifuge tube dropwise, mix well, incubate at 55 °C for 4 h; Chimpanzee adenovirus genome was extracted with phenol: chloroform: isoamyl alcohol (25: 24: 1) and chloroform: isoamyl alcohol (24: 1). The upper water phase was extracted by adding 1/10 volume of 3 mol/L NaAc and 2 times volume of absolute ethanol at-20 °C for 30 min, centrifuging at 4 °C at 12,400 r/min for 5 min, slowly discarding the supernatant, naturally drying the residual alcohol, and then adding 50 μL Elution Buffer to dissolve DNA.

The genomic DNA sequence of chimpanzee adenovirus was confirmed by agarose gel electrophoresis, and the results are shown in Fig. 3, where M: 15000 bp Marker; 1, 2: Genomic DNA of chimpanzee adenovirus, and the sequence larger than 15000bp is the genome of chimpanzee adenovirus.

**EXAMPLE 2 classification of novel chimpanzee adenoviruses**

The chimpanzee adenovirus genome DNA obtained in Example 1 was sequenced in the whole genome, and Shenggong Bioengineering (Shanghai) Co., Ltd. was responsible for determining the final virus genome sequence. The chimpanzee adenovirus was deposited in China Typical Culture Collection Center, classified and named as orangutan adenovirus sAd-AY01, and the preservation number was CCTCC NO: V202120. The genome of orangutan adenovirus sAd-AY01 is 36687bp.

Human adenoviruses belong to adenoviridae and mammalian adenoviruses. Up to now, 52 serotypes of human adenoviruses have been isolated, belonging to a ~ G7 subgenera. At present, virus isolation technology, antibody detection technology and molecular biology technology (such as polymerase chain reaction, restriction fragment length polymorphism analysis technology, in situ hybridization technology, real-time fluorescence quantitative PCR technology) are mainly used in the diagnosis and typing of adenovirus infection.

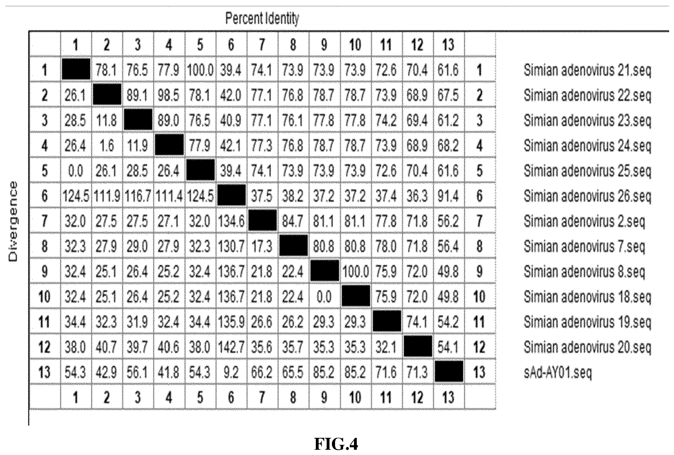

The invention is based on the comparison of the sequence of HVR1-6 of the hypervariable region of the hexon gene with the sequence of HVR1-6 of the known serotype in the database (reference: Rika MO, Yasushi S, Tsunetada K, et a 1. Quantitative Detection and Rapid Identification of Human Adenoviruses.J Clin Microbio1.2007. 4 5: 9 5 8. 967.). The comparison results are shown in fig. 4, where 1 ~ 12 represent HVR1-6 sequences of 12 known serotypes, and 13 represents HVR1-6 sequence of orangutan adenovirus sAd-AY01. From Fig. 4, it can be seen that the homology between HVR1-6 sequence of orangutan adenovirus sAd-AY01 and some known serotypes HVR1-6 sequences is between 49.8% and 67.5%. Based on the sequence of HVR1-6 in the hypervariable region of its hexon gene, it is compared with the known chimpanzee adenovirus partial serotype HVR1-6 sequence, monkey adenovirus partial serotype HVR1-6 sequence and human adenovirus partial serotype HVR1-6 sequence in genebank, the homology is between 49% and 91%. The phylogenetic tree was constructed by DNAstar software (Figure 5-2), and the homology and difference of sequences were analyzed by ClustalW calculation method. The adenovirus sequence with the largest homology was part of the gene of Pan troglodytes adenovirus clone 17.2 major capsid protein gene (GenBank: MF176134.1), and the largest homology was 91.63%. Finally, it was determined that the chimpanzee adenovirus was a new adenovirus that had not been discovered. The sequence of HVR1-6 of the new chimpanzee adenovirus are as below:

Seq ID NO.9:HVR1: ATAACCAAAGACAATGGAACTGATAAG;
Seq ID NO.10:HVR2: GAGTCAGGGGGTGAAAGCAAGAAA
Seq ID NO.11:HVR3: CATGATACTATTGGAGCTGAAGACAAG
Seq ID NO.12:HVR4: AAGGACGATGGCACTACT
Seq ID NO.13:HVR5: AGTCAGCAAGCTAGT
Seq ID NO.14:HVR6: GGCACTGATGAAACAAGT

**EXAMPLE 3 Construction of a Novel Chimpanzee Adenovirus (psAd) Vector Containing Fluorescent Labels**

The chimpanzee adenovirus (sAd) genome is a linear DNA larger than 36kb in length, which is difficult to edit effectively. Therefore, it is necessary to connect the replication-related elements in bacteria, such as replicon (pACYC) and resistance gene (kanamycin), to the chimpanzee genome to form a circular plasmid.

Therefore, it is necessary to construct a circular plasmid with a size of about 6000bp from replicon (pACYC), resistance gene (kanamycin), left arm (~ 1400bp) and right arm (~ 1400bp) of chimpanzee genome, and then transform it into BJ5183 competence together with chimpanzee adenovirus genome after linearization, and carry out homologous recombination to form a circular plasmid of chimpanzee, which can make it replicate effectively in bacteria. At the same time, in order to monitor the transfection efficiency of plasmids in cells, green fluorescent expression frames (RSV promoter, EGFP, BGHpolyA) were added. After transfection into cells, the transfection efficiency of large vectors was judged by the amount of fluorescence in TP0 generation.

3.1 Construction of adenovirus genomic DNA cyclic shuttle plasmid psAd-shuttle

Primer design of chimpanzee adenovirus sAd-AY01 left arm, right arm and bacterial element
PACYC-F: tttgaggtatattattgatgatgttaattaTTGGTTCCACTTGAGCGTCAGAC(SEQ ID:18)

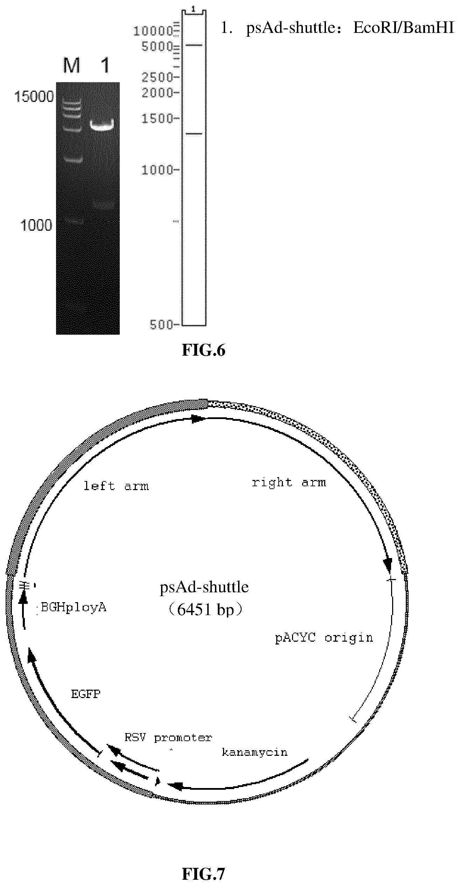

BGHpolyA-R:tttgaggtatattattgatgatgttaattaaAGACGAAAGGGCCTCGTTTGTA(SEQ ID: 19)
Left arm-F: catcatcaataatatacctcaaac(SEQ ID:20)
Left arm-BamHI-R:
CTTTGCTGGGAAGCCACCGTGGGAGGATCCAAGTAAACACACATGTGGCA(SEQ ID:21)
Right arm-F: TCCACGGTGGCTTCCAGCAA(SEQ ID:22)
Right arm-R: catcatcaataatatacctcaaac(SEQ ID:23)
Gene synthesis bacterial element, replicon (pACYC), resistance gene (kanamycin), green fluorescent expression frame (RSV promoter, EGFP, BGHpolyA);
3 fragments of left arm, right arm and bacterial element were amplified by PCR;
It was cloned seamlessly by using BM smealess cloning kit;
(5) Kanamycin resistance was screened, and the correct colonies were selected for enzyme digestion identification. The results are shown in Fig. 6, in which M: 15000bp Marker; 1: psAd-shuttle BamHI-EcoRI digestion. As can be seen from fig. 6, the shuttle plasmid psAd-shuttle was successfully constructed, and the map of the constructed shuttle plasmid psAd-shuttle is shown in fig. 7.

3.2 Chimpanzee adenovirus vector plasmid psAd was obtained by homologous recombination of shuttle plasmid psAd-shuttle and chimpanzee adenovirus genome

① BamHI linearized shuttle plasmid psAd-shuttle, and the enzyme digestion system was as follows:
Shuttle plasmid psAd-shuttle 3 $\mu$ g; BamHI 2 $\mu$ L; Buffer cutsmart 4 $\mu$ L; Replenish water to 40 $\mu$ 1. The reaction condition is 37 °C, 1h; 65 °C, 20min;
(2) Dephosphorylation of enzyme digestion products

Reaction system: Enzyme digestion reaction solution 40 $\mu$ L; Dephosphorylase 1 $\mu$ L; Dephosphorylated buffer 5 $\mu$ L; Replenish water to 50 $\mu$ L. The reaction condition is 37 °C, 1h; 65 °C, 5min inactivation;
Used OMEGA Ultra-Deep Gel Extraction Kit to recycle glue;
BJ5183 competent cells were co-transformed with 100ng of purified shuttle plasmid and 100ng of adenovirus genomic DNA. The transformed products were coated with LB plate containing Kan and cultured at 37 °C for 12 ~ 16h;

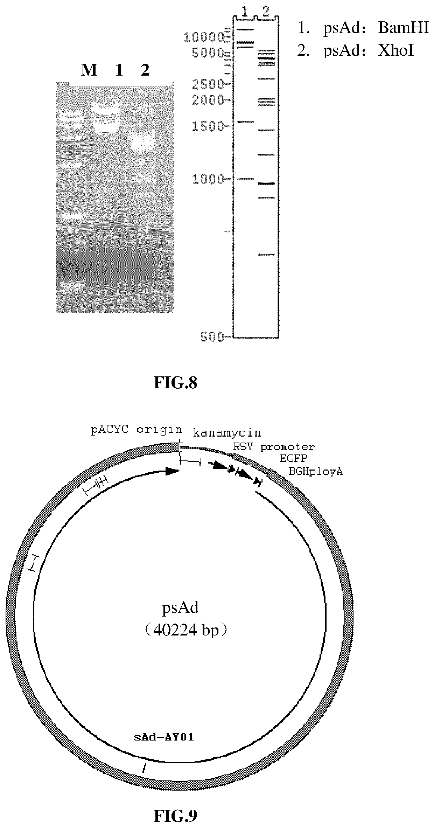

The colonies were cultured in 5mL LB liquid medium containing Kan for 12 ~ 16h at 37 °C, and the plasmid was extracted for enzyme digestion verification. The results are shown in Fig. 8, in which M: 15000bp Marker; 1: psAd BamHI digestion; 2: psAd XhoI digestion; As can be seen from fig. 8, a new chimpanzee adenovirus psAd plasmid containing fluorescent markers was successfully constructed, and the constructed plasmid map is shown in fig. 9.

**EXAMPLE 4 Construction of a novel replication-deficient chimpanzee adenovirus vector psAd E1 E3**

4.1 The E1 region of chimpanzee adenovirus vector psAd was excised by CRISPR/cas9 method to obtain the vector plasmid psAd E1 with E1 knockout, and the digestion site PsiI was introduced

① Selection of target sequence of target gene E1 CRISPR

The target sequence of E1 gene knockout was determined by using Thermofisher.com/crisprdesign software.

E1A: TCATTCACCGCCTCCTCGT(SEQ ID:24);
E1B: ggacctgcgacccgatcatt(SEQ ID:25);
DNA Amplification of sAd-E1A-gRNA and sAd-E1B-gRNA

1) DNA template design of sAd-E1A-gRNA

5                                                                                                                   '-

TAATACGACTCACTATAGATTCACCGCCTCCTCGTTTTTTAGAGCTAAAATAGCAAGGC

TAGTCCGTATCAACTTGAAAAGTTTT-3'(SEQ ID:26)

2) DNA template design of sAd-E1B-gRNA

5'-Designing upstream and downstream primers of DNA template for amplification of sAd-E1A-gRNA and sAd-E1B-gRNA

sAd-E1A-gRNA-Forward: TAATACGACTCACTATAG ATTCACCGCCTCCTC(SEQ ID:27)
sAd-E1A-gRNA-Reverse: TTCTAGCTCTAAAAC AACGAGGAGGCGGTGAAT(SEQ ID:28)
sAd-E1B-gRNA-Forward: TAATACGACTCACTATAGacctgcgacccgatc(SEQ ID:29)
sAd-E1B-gRNA-Reverse: TTCTAGCTCTAAAACaatgatcgggtcgcaggt(SEQ ID:30)

4. According to the instructions of Invitrogen ™ GeneArt ™ Precision gRNA Synthesis Kit, DNA templates for amplifying sAd-E1A-gRNA and sAd-E1B-gRNA are obtained, and sAd-E1A-gRNA and sAd-E1B-gRNA are obtained by in vitro transcription, and purified to obtain gRNA. The sequence after in vitro transcription is as follows:

sAd-E1A-gRNA:

GAUUCACCGCCUCCUCGUUGUUUUUAGAAAUAGCAAGUUAAAAUAAGGCUAG
UCACAACUUGAAAAAGUGUUU(SEQ ID:31)

sAd-E1B-gRNA:

GaccUgcgacccgaUcaUUGUUUUUAGAAUAGCAAGUUAAAAUAAGGCUAGUUUCA
ACUUGAAAAAAGGUGUUU(SEQ ID:32)

⑤ CRISPR/Cas9 "digestion" psAd

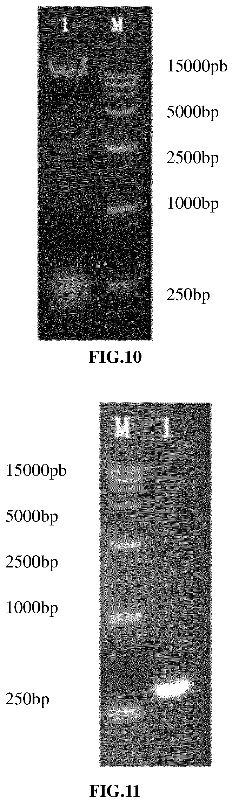

The vector plasmid psAd was obtained by digesting example 3 with sAd-E1A-gRNA, sAd-E1B-gRNA and cas9. The reaction system was Cas9 protein 3 μ g, sAd-E1A-gRNA 3ug, sAd-E1B-gRNA 3ug, psAd vector plasmid 3ug, NEB buffer 3.5 uL and supplemented water to 50uL.
The reaction was incubated overnight at 37 °C and detected by agarose gel electrophoresis. The results are shown in Fig. 10, where M: 15000bp Marker; 1: psAd "digested" E1 with cas9. As can be seen in Fig. 10, the CRISPR/Cas9 "digested" psAd plasmid was successfully obtained and the vector was purified using the Axygen Gel Recovery Kit.

⑥ Amplify the HRPT sequence inserted into E1 region, which contains PsiI cleavage site Amplification primers:

sAd-E1-HRPT-F: atgtgcccgagaaccccccaaccttgcatttctcagtcctaaac(SEQ ID:33)
sAd-E1-HRPT-R: cccgttgcaggacaacaccaaatcactttgcatatagtatacatg(SEQ ID:34)

The amplified sequence is:

cttgcatttctcagtcctaaacagggtaatgggctggggctgaatcacatgaaggcaaggtcagattttttattattatgcacatctagctt
gaaaattttccattaagtcaattacagtgaaaaacctcacctggtattgaatgcttgccttgtatgtctggctattctgtgtttttattttaaaattataa
tatcaaaatatttgtgttataaaatattctaactatggaggccataaacaagaagactaaagttctctcctttcagccttctgtacacatttcttctca
agcactggtttatgcatgtatactatatgcaaaagtg(SEQ ID:35)

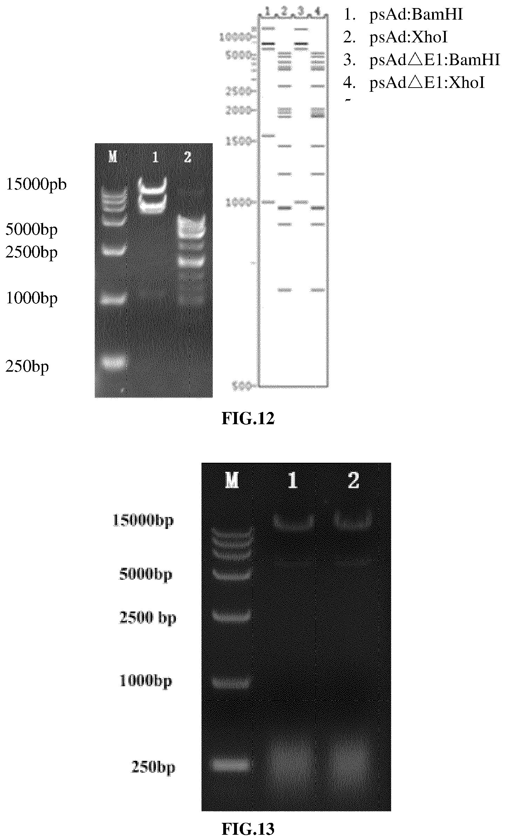

PCR amplified fragment, as shown in Fig. 11, where: M: 15000bp Marker; 1: HRPT fragment; The fragment was purified using Axygen gel recovery kit.
⑦ Using BM smealess cloning kit of Beijing Bomade Gene Technology Co., Ltd. to connect the vector with the fragment; The identification results after enzyme digestion are shown in Fig. 12, and the chimpanzee adenovirus vector psAd △E1 with E1 gene deletion was successfully obtained.

4.2 The vector plasmid psAd △E1△E3 was obtained by excising the E3 region of psAd △E1 adenovirus by CRISPR/cas9

① Selection of target sequence of target gene E3 CRISPR

The target sequence of E1 gene knockout was identified using the Thermofisher.com/crisprdesign software GeneArt ™ CRISPR Search and Design tool (thermofisher.com/crisprdesign)

sAdE3A: AGCGGCAGGGATACAAGTCC(SEQ ID:36);
sAd E3B: GGGAAATCACCCTCAAGCT(SEQ ID:37);
DNA Amplification of sAd-E3A-gRNA and sAd-E3B-gRNA

1) DNA template design of sAd-E3A-gRNA

5                                                                                          '-
TAATACGACTCACTATAGCGGCAGGGATACAAGTCCGTTTAGAGCTAAAATAGCAAGG

GTTT-3'(SEQ ID:38)

2) DNA template design of sAd-E3B-gRNA

5                                                                                          '-
TAATACGACTCACTATAGGAAATCACCCTCAAGCTGTTTTAGAGCTAAAATAGCAAGT

TAAAATAGGGCTAGTCCGTATCAACTTGAAAGGGGCACCGAGGGGGCACCGAGGGT

GGTGTGTTTTTT-3'(SEQ ID:39)

Designing upstream and downstream primers of DNA template for amplification of sAd-E3A-gRNA and sAd-E3B-gRNA

sAd-E3A-gRNA-F: TAATACGACTCACTATAGCGGCAGGGATACAAG(SEQ ID:40)
sAd-E3A-gRNA-R: TTCTAGCTCTAAAACGGACTTGTATCCCTGCCG(SEQ ID:41)
sAd-E3B-gRNA-F: TAATACGACTCACTATAGGAAATCACCCTCAA(SEQ ID:42)
sAd-E3B-gRNA-R: TTCTAGCTCTAAAACAGCTTGAGGGTGATTTCC(SEQ ID:43)

4. According to the instructions of Invitrogen ™ GeneArt ™ Precision gRNA Synthesis Kit, DNA templates for amplifying sAd-E3A-gRNA and sAd-E3B-gRNA are obtained, and sAd-E3A-gRNA and sAd-E3B-gRNA are obtained by in vitro transcription, and purified to obtain gRNA. The sequence after in vitro transcription is as follows:

sAd-E3A-gRNA:

GCGGCAGGGAUACAAGUCCGUUUUAGAAUAGCAAGUUAAAAUAAGGCUAGU

CCGUUAUCAACUUGAAAAAGAGUGUUU(SEQ ID:44)

sAd-E3B-gRNA:

GGGAAAUCACACCCUCAAGCUGUUUUAGAAAUAGCAAGUUAAAAGGUUAUC

ACAACUUGAAAAAGUGUUU(SEQ ID:45)

⑤ CRISPR/Cas9 "digests" E3 of psAd△E1

The psAd△E1 plasmid was digested with sAd-E3A-gRNA, sAd-E3B-gRNA and cas9. The reaction system was Cas9 protein 3ug, sAd-E3A-gRNA 3ug, sAd-E3B-gRNA 3ug, psAd△E1 vector plasmid 3ug, NEB buffer 3.15 $\mu$ L, and water supplementation to 50 $\mu$ L.

Enzyme digestion reaction was incubated at 37 °C overnight. The carrier was purified using Axygen Gel Recovery Kit. The results of enzyme digestion are shown in Fig. 13: M: 15000bp Marker; 1, 2: psAd E1 "digested" the product using cas9.

The excised pVIII and U-exon regions were amplified by PCR, and the glue was recovered; The amplification primers are:

E3A-F: AGGAGCGGCAGGGATACAAG(SEQ ID:46)
E3A-R: TCAATCGTAGCCGTCCACCG(SEQ ID:47)
E3B-F: AGTCGGTGGACGGCTACGATTGATCACCCCCTTATCCCAGTGAAAT(SEQ ID:48)
E3B-R: CGAGGTCCACCCCCTCC(SEQ ID:49)

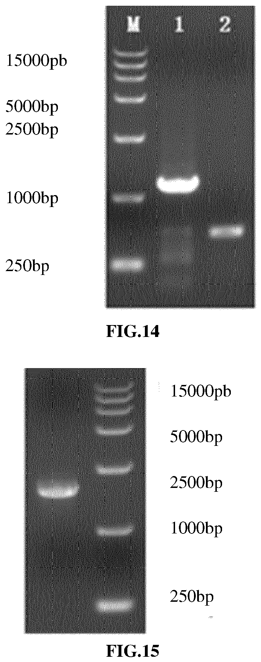

The amplified fragments are:
E3A-F and E3A-R were used as a pair of primers to amplify partial fragments of pVIII; E3B-F and E3B-R were used as a pair of primers to amplify partial fragments of U-exon. As shown in Fig. 14, where M: 15000bp Marker; 1: Partial fragment of PVIII; 2: U-exon partial fragment.

The pVIII-U-exon fragment was amplified by E3A-F and E3B-R fusion PCR, and the glue was recovered; As shown in fig. 15, the final fragment is a 1775bp fusion fragment.

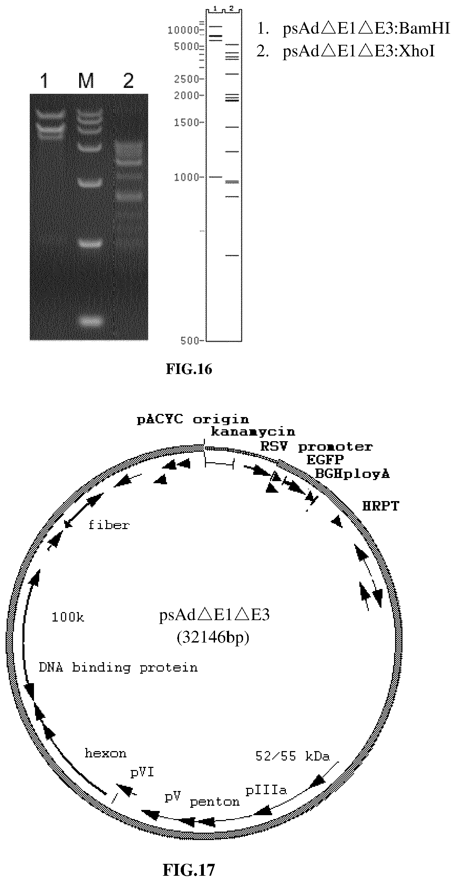

8 Using BM smealess cloning kit of Beijing Bomade Gene Technology Co., Ltd. to seamlessly clone the vector after cas9 "enzyme digestion" in step 5 and the pVIII-U-exon fusion fragment in step 7; Transforming to DH5 $\alpha$ competence, screening kanamycin resistance, culturing overnight at 37 °C, picking colonies, extracting plasmids, and digesting with BamHI and XhoI; As shown in Figure 16: M: 15000bp Marker; 1: psAd E1 E3 BamHI digestion; 2: psAd E1 E3 XhoI digestion; The picture on the right shows the enzyme digestion simulation of psAd E1 E3 plasmid. The results were correct, and the plasmid psAd E1 E3 was successfully constructed. The plasmid map is shown in Figure 17.

**EXAMPLE 5 Construction of a new chimpanzee adenovirus vector psAd△E1△E3 shuttle plasmid pSsAdEI and a shuttle plasmid pSsAdE1-S expressing Novel Coronavirus S gene**

The new chimpanzee adenovirus vector is 35kb long and has many restriction sites, so it is difficult to operate directly on the vector. It is necessary to construct its special shuttle plasmid and integrate the foreign genes to be expressed into the adenovirus vector by homologous recombination.

S protein is the main antigen protein of Novel Coronavirus, which will cause the main immune response in the process of infection. Therefore, COVID-19 vaccine, a new chimpanzee vector, chose S gene of COVID-19 as the expressed foreign gene. Therefore, it is designed to use CMV promoter to drive expression in E1 region, which can produce a large number of antigens and cause strong immune response.

5.1 Construction of a new shuttle plasmid pSsAdEI for chimpanzee adenovirus vector E1 region

To construct the shuttle plasmid of chimpanzee adenovirus vector, plasmid-related elements (replicon puc origin, resistance gene, eukaryotic system exogenous gene expression frame CMV-MCS-SV40 polyA) and the left arm and right arm of E1 region were connected in a suitable sequence.

① PCR amplified the left arm and right arm of chimpanzee E1 region to replicate related elements, resistance genes and CMV-MCS-SV40 polyA. The template of left arm and right arm was chimpanzee adenovirus vector, and the template of replicating related elements and resistance genes and CMV-MCS-SV40 polyA was obtained by synthesis.

The primer design is as follows
Left-arm-F: TGGATCGGTGATCACCGATCCCGTTTCAAAGTGTAGATCTGACTC(SEQ ID:50)
Left-arm-R: GTTAGCTCACTCATTAGGCACCCttaattaacatcatcaataatatacc(SEQ ID:51)
Puc-F: GGGTGCCTAATGAGTGAGCTA(SEQ ID:52)
Amp-R: GACGTCAGGTGGCACTTTTC(SEQ ID:53)
Right-arm-F: CCCGAAAAGTGCCACCTGACGTCTCCGACCAGCCGGATGAGC(SEQ ID:54)
Right-arm-R: CCCCGTAATTGATTACTATTAATAGAGTGAGTAGTGTTGGGG(SEQ ID:55)
CMV-F: TTAATAGTAATCAATTACGGGGT(SEQ ID:56)

SV40-R: CGGGATCGGTGATCACCGAT(SEQ ID:57)

PCR amplification system: left-arm-F and left-arm-R were used as a pair of primers to amplify the left arm of the shuttle plasmid, puc-F and Amp-R were used as a pair of primers to amplify the replicon and resistance genes needed by the plasmid, right-arm-F and right-arm-R were used as a pair of primers to amplify the right arm of the shuttle plasmid, and CMV-F and SV40-R were used as a pair of primers to amplify the foreign gene expression frame CMV-MCS-SV40 polyA (Seq ID NO. 16).

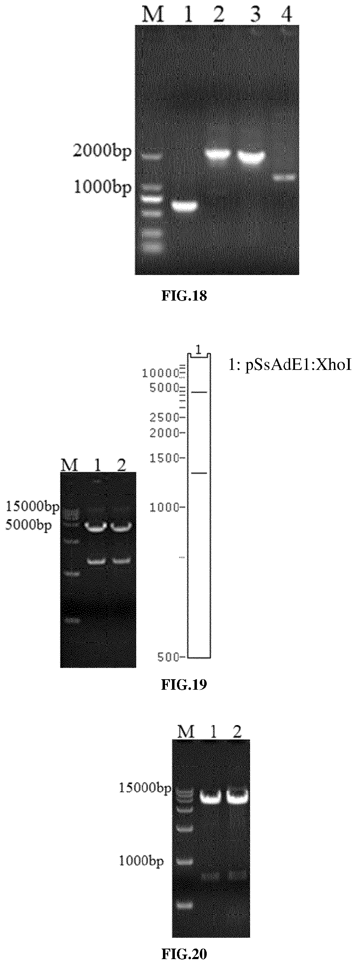

The amplification results are shown in Fig. 18: M: 2000 bp Marker; 1 is the fragment left arm; 2 is the fragment puc& 3 is the fragment right arm; 4 is fragment CMV-MCS-SV40.

The above four fragments were purified by Axygen gel recovery kit; 3. Using BM smealess cloning kit of Beijing Bomade Gene Technology Co., Ltd. to connect the four fragments;

Junction system: left arm, puc & amp, right arm, CMV-MCS-SV40 fragment 2ul; 2 × measure 10ul; Replenish water to 20ul. Reaction conditions: 50 °C, 40min. Transformed DH5 $\alpha$ competent cells were coated on LB solid medium (ampicillin-resistant) and cultured overnight at 37 °C.

After screening, the extracted plasmid was digested and identified; Results As shown in fig. 19, the shuttle plasmid pSsAdEl was successfully obtained.

5.2 Adding WPRE Element to Shuttle Plasmid to Obtain pSsAdEl-WPRE Element WPRE can keep the mRNA stable and increase the viral yield

① PCR amplification of WPRE element
Primer design
WPRE-NotI-F: ATATCCAGCACAGTGGCGGCCGGGCCGCTCGACAATCAACCTCTGGATTA(SEQ ID:58)
WPRE-NotI-R:

TAAACGGGCCCTCTAGACTCGAGGCGGCCGCCGGGGGGGGGGGGGGGGGGGGGGGGG

GGGGGGGGGCGCCA(SEQ ID:59)

PCR amplification of WPRE fragment
(3) NotI enzyme digestion of shuttle plasmid pSsAdEl, and enzyme digestion reaction system: vector pSsAdEl, NotI 1ul; 10 × cutsmart buffer 5ul; Replenish water to 50ul.

The WPRE and the digested pSsAdEl gum were recovered and purified;

It was cloned seamlessly with BM Smealess cloning kit;
Reaction system: pSsAdE1-UCOE0.65, WPRE fragment 2ul; 2 × BM smealess10ul; Replenish water to 20ul. Reaction conditions: 50 °C, 40min.

⑥ Transformed DH5 $\alpha$ competent cells, coated on LB solid medium (ampicillin resistance), cultured overnight at 37 °C, screened, extracted plasmid digested and identified; As shown in Figure 20: M: 15000 bp Marker; 1, 2 were identified by pSsAdEl-WPRE NotI enzyme digestion; The pSsAdEl-WPRE component was successfully built.

5.3 Construction of shuttle plasmid pSsAdE1-WPRE-S expressing complete S protein gene of COVID-19 in Novel Coronavirus

① The COVID-19 S protein gene was amplified by PCR, and the primers were designed as follows:

S-EcoRV-F: TCCCTGATCACTGCAGAATTCGATATCgccaccATGTTCGTTTTCTGGTGCTG(SEQ ID:60)
S-WPRE-HindIII-R: ATTGTCGAGCGGCCGCAATCGATAAGCTTtcaGGTGTAGTGCAGCTTCAC(SEQ ID:61)

② pSsAdEl-WPRE was digested by EcoRV and HindIII, and the enzyme digestion reaction system was pSsAdEl-WPRE, EcoRV and HindIII 1ul; 10 × cut smart buffer 5ul; Replenish water to 50ul.

Collecting and purifying S and pSsAdEl gum after enzyme digestion;

It was cloned seamlessly by using BM Smealess cloning kit;

Reaction system: pSsAdEl-WPRE, S fragment 2ul; 2X BM smealess 10ul; Replenish water to 20ul. Reaction conditions: 50 °C, 40min.

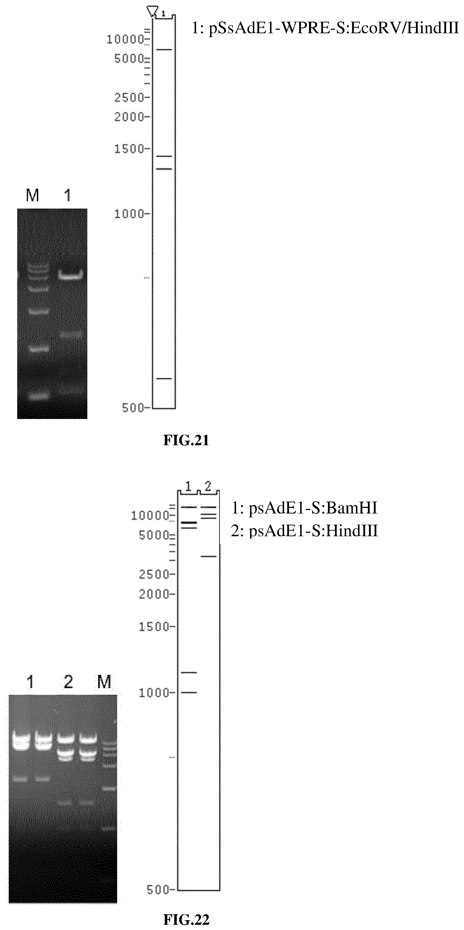

(5) Transformed DH5 α competent cells, coated on LB solid medium (ampicillin resistance), cultured overnight at 37 °C, screened, extracted plasmid digested and identified; As shown in Figure 21: M: 2000 bp Marker; 1, 2 were identified by pSsAdE1-WPRE-S EcoRV & HindIII enzyme digestion; The results were correct, and the shuttle plasmid pSsAdE1-WPRE-S was successfully constructed.

**EXAMPLE 6 homologous recombination to obtain a new recombinant chimpanzee adenovirus vector psAd-S**

6.1 Homologous recombination of shuttle plasmid pSsAdE1-WPRE-S and adenovirus vector plasmid psAd △E1△E3

1) PacI and PsiI digested the shuttle plasmid pSsAdE1-WPRE-S and the adenovirus vector plasmid psAd △E1△E3. The enzyme digestion system was as follows:

A, shuttle plasmid pSsAdE1-WPRE-S 3 μ g; PacI 2 μ L; Buffer cutsmart 4 μ L; Replenish water to 40 μ 1.
B, adenovirus vector plasmid psAd△E1△E3 3ug; PsiI 2 μ L; Buffer cutsmart 4 μ L; Replenish water to 40 μ 1.

The reaction condition is 37 °C, 1h; 65 °C, 20min inactivation.

2) Dephosphorylation of digested products

Reaction system: Enzyme digestion solution 37.5 μ L; Dephosphorylase 1 μ L; Dephosphorylated buffer 5 μ L; Replenish water to 50 μ L. The reaction condition is 37 °C, 1h; 65 °C, 5min inactivation.

3) Vectors and fragments were recovered using the OMEGA Ultra-Deep Gel Extraction Kit.

4) Competent BJ5183 cells were co-transformed with 100ng of purified shuttle plasmid and 100ng of purified adenovirus vector. The transformed products were coated with LB plate containing Kan and cultured at 37 °C for 12 ~ 16h.

5) The colonies were cultured in 5mL LB liquid medium containing Kan at 37 °C for 12 ~ 16h, and the plasmids were extracted for XhoI digestion.

6) Transforming the positive plasmid into DH5a competence, picking a colony in 5mL LB liquid medium containing kanamycin, shaking culture at 37 °C for 12 ~ 16h, and extracting the plasmid for HindIII and BamHI digestion verification again. As shown in Fig. 22: M: 15000 bp Marker; 1: psAd-S HindIII digestion identification; 2: psAd-S BamHI digestion identification; successfully constructing a new recombinant chimpanzee adenovirus vector psAd-S.

**EXAMPLE 7 Packaging of the novel recombinant chimpanzee adenovirus psAd-S**

The psAd-S vector plasmid was packaged using 293 cells (purchased from ATCC, lot number 70009859) as follows:

① Preparation of 293 cells: The day before transfection, the cells to be transfected were prepared and inoculated into a 6-well plate, 0.5 × 106/well, and cultured at 37 °C and 5% CO2 for 24 hours. On the day of transfection, the confluence rate of cells was 40-50%.

(2) Linearization of plasmid psAd-S: The plasmid to be transfected was digested with I-sceI enzyme, incubated at 37 °C for 40min, and then inactivated at 65 °C for 20min.

Transfection: The linearized 2 μ g plasmid and PEI were diluted with 100 μ L serum-free medium respectively; Plasmid diluent was added to PEI diluent, and then absorbed repeatedly for 5 times or swirled for 10 seconds, and then incubated at room temperature for 10 minutes to form transfection complex. During incubation, the cell culture medium was gently sucked from the culture plate, and 2mL of fresh growth medium was added. After 10 minutes, the transfection complex was added to the cells changed from fresh medium.

Cell culture: 293 cells after transfection were cultured in 37 °C, 5% CO2 incubator for 72-96 hours; After 72-96 hours of viral plasmid transfection, 6-well plate cell suspension was collected and placed in 15ml centrifuge tube, i.e. TP0.

(5) Continuous inoculation: freeze-thaw the collected cell suspension at-80 °C for 3 times, centrifuge at 4 °C and 2000g for 10 minutes, infect 293 cells with 500 μ L supernatant (293 cells need to be prepared one day in advance), incubate at 37 °C and 5% CO2 for 60 minutes, supplement 2mL FBS culture medium, culture at 37 °C and 5% CO2 for 72 hours, and collect cell suspension, namely TP1; Repeat the previous steps to collect cell suspension, namely TP2. Continue to inoculate until the cells appear pathological changes.

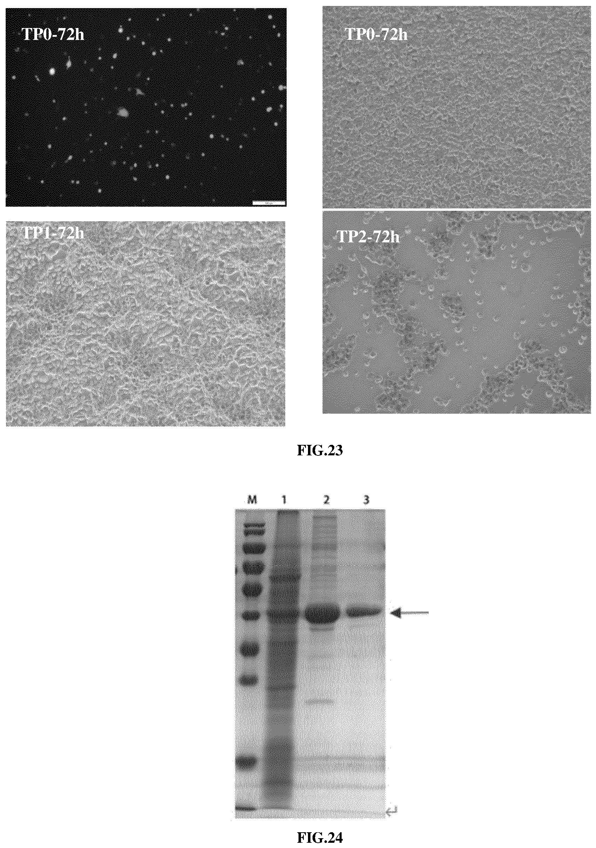

⑥ Cytopathic changes: When 293 cells were cultured from TP0 to TP2, the cells gradually became pathological changes until 293 cells became completely pathological changes. The cytopathic changes caused by TP0 to TP2 are shown in Fig. 23, respectively, and TP2 has completely changed.

**Example 8 titer detection of recombinant new chimpanzee adenovirus psAd-S vaccine**

8.1 HVR antigen protein of hypervariable region of hexon protein of new chimpanzee adenovirus (hexon)

① Construct prokaryotic expression pET28b-sAd-HVR, and pET28b plasmid was donated by Beijing Bomade Gene Technology Co., Ltd.;
Transformation of PET-28a-sAd-HVR plasmid into BL21 (DE3) strain;
(3) Overnight expression of IPTG at 24 °C;
Harvesting the bacteria liquid, centrifuging, and ultrasonic crushing the bacteria precipitate with PBS re-suspension;
Ultra-speed centrifugation to obtain supernatant;
(5) purifying the supernatant by NI column to obtain sAd-HVR protein, and the result is shown in fig. 24, in which M: protein Marker; 1. supernatant of sAd-HVR bacteria liquid; 2: purified sAd-HVR protein; 3. purified sAd-HVR protein diluent;
8.2 Preparation of HVR antibody against hexon protein (hexon) hypermutation region of chimpanzee adenovirus Dissolve HVR protein in normal saline;
2. Mix with equal volume Freund's complete adjuvant by double push method (antigen concentration is about 0.1 mg/ml);
BALB/C mice were immunized by subcutaneous multi-point injection with 0.2 ml;
④ After 3 weeks, the same amount of antigen and incomplete Freund's adjuvant were mixed evenly and immunized;
(5) After 4 weeks, the above methods were used to strengthen immunization once a week (incomplete Freund's adjuvant) for 3 times in total;
⑥ Three days after the last immunization, the serum titer was measured by cutting off the tail. If the expected purpose was achieved, the antiserum could be collected by bloodletting.
⑦ After indirect ELISA detection, the concentration of HVR antibody reached 1: 200,000, which met the conditions and was finally released;

8.3 Detection of titer with HVR antibody of new chimpanzee adenovirus

293 cells were prepared. The cells grew well in T75 culture flask, discarded the supernatant, washed with PBS, digested with 0.25% trypsin, then added 10ml DMEM fresh medium containing 10% fetal bovine serum to stop digestion, then blew and mixed evenly, inoculated into 6-well plate ($5 \times 10^5$/ml, 2ml per well), and stood in a 5% CO2 and CO2 incubator at 37 °C. After 24 hours, when the cells grew into monolayer cells, the culture medium was discarded, and the recombinant adenovirus was continuously diluted by $10^{-2} \sim 10^{-6}$ times with serum-free DMEM maintenance solution. Each dilution was inoculated into 2 wells with 250uL per well. After 1 hour of infection, the supernatant was discarded, and the complete culture medium was supplemented, and then the culture was static in a 5% CO2 and CO2 incubator at 37 °C. After 24 hours, the supernatant was discarded and the cells were washed with PBS, 1mL per well. After discarding PBS, the cells were fixed with 1mL cold formaldehyde at room temperature for 10min. After discarding formaldehyde, the cells were washed with PBS, 1mL per well, and then incubated with chimpanzee adenovirus HVR antibody, 1ml per well. After 1h at room temperature, the cells were washed with PBS again, 1mL per well, and the sheep anti-rabbit-FITC second antibody was incubated. After 1h at room temperature, the supernatant was discarded and the cells were washed with PBS again, and counted under fluorescence microscope (200 times, 10 continuous visual fields). Calculation: Virus titer (FFU/mL) = average $\times 10^{13} \times 4 \times 10^{(-n)}$. The FFU of psAd-S virus is $5 \times 10^8$ FFU/ml.

8.4 Comparison of titers of different adenovirus vectors

The adenovirus stored by our company and HAd26, HAd5 and HAd28 produced in the market are used to detect the virus titer. The results are shown in Table 1. sAd-AY01 is a new chimpanzee adenovirus provided by the invention, and its titer is the highest, which can reach $5 \times 10^8$ FFU/ml, providing the most basic guarantee for the industrial production of chimpanzee adenovirus vaccine.

Table 1. Comparison of titers of adenovirus vectors

| Virus type | SAd-AY01 | HAd 5 | HAd26 | HAd 28 | Simian Adenovirus 40.1 |
|---|---|---|---|---|---|
| Titer FFU/mL | $5 \times 10^8$ | $1 \times 10^8$ | $5 \times 10^7$ | $4 \times 10^7$ | $1 \times 10^8$ |

**EXAMPLE 9 Detection of stability of recombinant new chimpanzee adenovirus psAd-S vaccine**

The 293 cells were prepared. The cells grew well in T75 culture flask, discarded the supernatant, washed with PBS, digested with 0.25% trypsin, then added 10ml of DMEM fresh medium containing 10% fetal bovine serum to stop digestion. Then, the 293 cells were planted in a 6-well plate ($5 \times 10^5$ cells/ml, 2ml/well), incubated at room temperature for 1 hour to make them adhere to the wall. After incubation, the adherence degree was examined under microscope. The infection was carried out with psAd-S virus particles, and the titer of infection was 2MOI/well. After the 293 cells became pathological changes 48 hours later, the cells were collected, frozen and thawed repeatedly for 3 times, then centrifuged at 2000g, and the supernatant was collected. FFU was detected in the collected supernatant, and then the new 293 cells were re-infected until 30 generations. The virus fluids collected from the 5th, 10th, 15th, 20th, 25th and 30th generations were detected, and it was found that the virus genome was still intact, which indicated that replication-defective psAd-S virus could be stably packaged in 293 cells.

**Example 10 detection of psAd-S recovery mutation (RCA) in recombinant novel chimpanzee adenovirus**

The psAd-S virus RCA detection method is as follows:

1. Prepare psAd-S virus solution, measure its virus titer and determine the concentration of virus particles. The virus solution was mixed with 1% Universal Nuclease (virus solution with the concentration of 7.5-15 units/mL) to digest the DNA of the host cells, and the water bath was kept at 37 C for 40min. The virus particles were collected using a 300Kd ultrafiltration centrifuge tube after centrifugation at 1000g for 30min, followed by elution with $1\times$PBS. A260 was measured as the particle concentration $=A260\times1.1\times10^{12}$ VP/mL.

2. For virus infection, six-well plates of A549 cells were prepared, with each well cell being $2.5\times10^5$/ well. The medium was discarded and washed with PBS once. Adenovirus was inoculated with virus at $1\times10^9$vp/ well to infect A549 cells. Wild-type sAd-AY01 adenovirus was used as the control at 37 °C and 5% $CO_2$. After 1h, the virus solution was discarded and made up of 5% complete medium. The cells were cultured at 37 °C and 5% $CO_2$ for 48 h.

3. Immunostaining was performed, and the cell supernatant was discarded. The cells were surface washing cells in PBS, fixed with ice formaldehyde, placed at - 20 °C for 20min, and washed with $1\times$PBS for three times, each time for 5min. Then 2ml 1% BSA-PBS solution was added into each well, placed in a shaker, and incubated for 1 h. After the supernatant was discarded, adenovirus type 5 fluorescent antibody (1:500 dilution) was added and incubated for 1h, followed by washing with $1\times$PBS for three times, 5min each time.

RCA was calculated using the equation as observed under a 10-fold fluorescence microscope

$$\text{RCA}=(\text{average positive cell field})\times（374 \text{ field/well}）\times（\text{dilution factor}）)/ \text{ Total VPs in } 0.5\text{ml viral sample}$$

The judging standard was that the level of RCA was less than 1RCA/$3\times10^{10}$vp. After statistics, the RCA level of psAd-S is less than 1RCA/$3\times10^{10}$vp, which indicates that the replication-defective psAd-S virus prepared by the invention can be stably packaged in 293 cells and cannot be converted into wild type or has low probability of being converted into wild type.

**Example 11 detection of expression of recombinant novel chimpanzee adenovirus psAd-S protein**

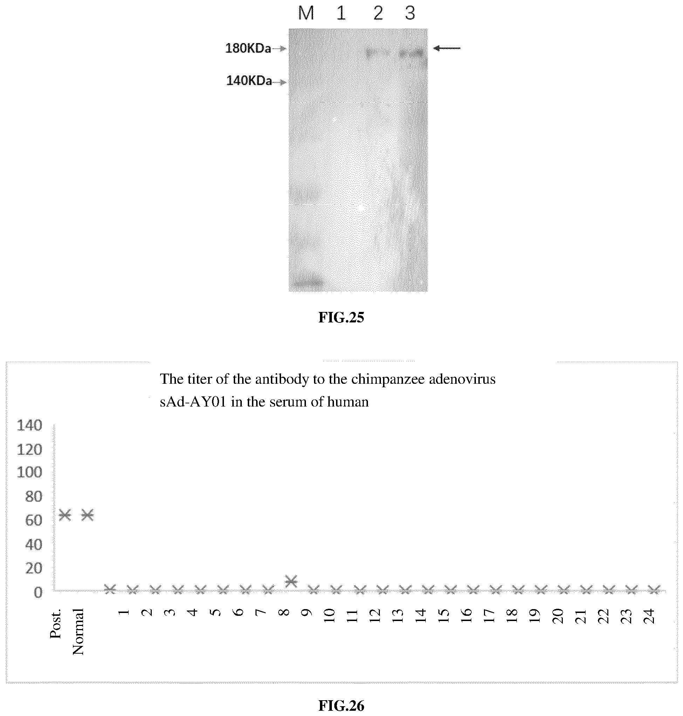

The 293 cells were prepared one day in advance and placed in a 12-well cell culture plate. The 293 cells were infected with psAd-S virus and the cells became diseased 48 hours later. All 1ml of cells were collected and washed with PBS for sample preparation for Western Blot test. Spike's RBD protein antibody was used as the secondary antibody to detect the target protein. The RBD protein antibody was purchased from Beijing Yiqiao Shenzhou Technology Co., Ltd. The results of Western Blot test are shown in Fig. 25: M: 180 kDa marker. 1, 293 blank cell control; 2, 3: a sample of 293 cells infected with pSAd-S virus; The results showed that the S protein expressed by the chimpanzee adenovirus vector could be efficiently expressed in 293 cells.

**Example 12 chimpanzee adenovirus pre-stored immune detection**

Neutralizing antibody against AdHu5 is common in the population, and about one-third of the population has been immunized against AdHu5 in adulthood, while the population does not have the antibody against the novel chimpanzee adenovirus. Therefore, injection of the novel chimpanzee adenovirus vector vaccine into human body will not be neutralized by pre-existing immunity, and sufficient antibody can be produced. The result can be verified by neutralize antibody experiments. The experimental design was shown in Table 1, and the experimental steps were as follows:

(1) One day before, 96-well plates were paved for the preparation of A549 cell suspension, so that the cell concentration was about $4.8\times10^5$ cells /mL (each 96-well plate was prepared according to $4.8\times106$ cells). The cells were added into 96-well cell culture plates with 100μl per well by 8-way guns in a 5% CO2 incubator at 37 C for static culture;

(2) On the second day, wild-type virus (sAd-AY01-TP5, Ad5-Wild-F2) was inoculated, and 1.5ml of sterilized EP tube was taken and diluted in accordance with 10-1, 10-2, 10-3, 10-4, 10-5, 10-6, 10-7, 10-8(900μl serum-free medium+100μl virus solution) at a multiple ratio. Note that Tip is replaced for each dilution, and it is mixed evenly on a vortex oscillator for 5s for standby.

(3) For cells in 96-well plate, 200μl tip was connected by vacuum pump to discard the supernatant, followed by cell washing with 1×PBS, and the supernatant was discarded again;

(4) Sensitized cells were treated with 100 L/well for 2h, the supernatant was discarded, and the complete medium supplemented with 5%FBS was added at 200μl/ well, 37 C and 5%CO2 for static culture. After the lesions were observed on days 5-7, the TCID50; was calculated according to the Reed-Muench method.

**Neutralization experiment**

(1) Human serum 1200μl/1.5EP tube and rabbit serum 500μl/1.5EP tube were separately packaged and inactivated in a water bath at 56 °C for 30min.

(2) Dilution of serum: 8-channel pipette sucks 110μl of serum-free F12K medium into a 96-well plate, where the first column and the seventh column are empty. After the serum is inactivated, 110μl of serum is taken into the first column and the second column, respectively. After the second column is mixed evenly, 110μl is taken into the third column, and diluted into the sixth column by analogy ratio. The last 110μl of diluent is discarded, and six dilutions of 2-1, 2-2, 2-3, 2-4, 2-5, 2-6 are total, as described above

(3) Dilution of virus: 50/100 μ L of 200 TCID was used to prepare the virus solution.

(4) Mix: virus and serum. The diluted virus solution was sucked into a 10cm plate, and 110μl virus solution was sucked into the diluted serum to be examined by eight rows of guns (110μl serum +110μl virus), 37°C, 5%CO2, 1 hour;

(5) After 2 hours, 200μl Tip was connected with a vacuum pump, the cell supernatant from the 96-well plate was discarded, and 200 μ L of cells washed with 1 × PBS were aspirated. Then 100μl of the above mixture was aspirated as the sensory cells. Two wells were parallel. A total of four wells (37 °Cand 5%$CO_2$) were used for each serum dilution, and the reaction lasted for 2 hrs.

(6) After 2 hours, connect 200μl Tip with vacuum pump, discard the above mixture, and supplement 200 μ L 5% FBS at 37°C and 5%CO2 for incubation. The results were judged on days 3-7, with the serum dilution causing 50% cytopathic effects as the neutralization titer.

(7) Yin, positive control and blank control were set in the experiment. The positive serum control this time was rabbit serum immunized with wild type sAd-A Y01 and Ad5.

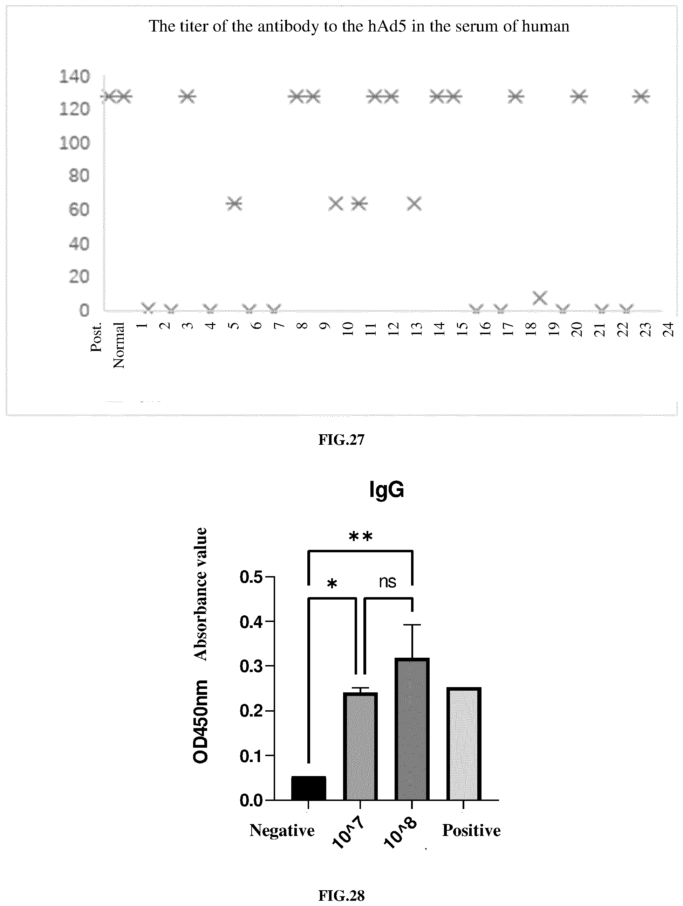

The neutralizing antibody experiments were conducted with chimpanzee adenovirus sAd-AY01 and human adenovirus type 5, respectively. The results showed that none of the dilutions of the positive serum sickness venom was pathological changes, and all of the dilutions of the negative serum sickness venom were pathological changes. The experiments were established. The specific results are shown in Table 2 and Table 3, Fig. 26 and Fig. 27, where Table 2 and Fig. 26 are pathological changes of sAd-AY01 neutralizing antibody experimental cells, and Table 3 and Fig. 27 are pathological changes of hAd5 neutralizing antibody experimental cells.

Table 2: Pathological Changes of 2: sAd-AY01 Neutralizing Antibody Experimental Cells

| sAd-AY01 | 1 :2 | 1 :4 | 1 :8 | 1 :16 | 1 :32 | 1 :64 |
|---|---|---|---|---|---|---|
| Positive sample | - | - | - | - | - | - |
| Inactive virus | + | + | - | - | - | - |
| Blank cell | - | - | - | - | - | - |
| Sample 1 | + | + | + | + | + | + |
| Sample 2 | + | + | + | + | + | + |
| Sample 3 | + | + | + | + | + | + |
| Sample 4 | + | + | + | + | + | + |
| Sample 5 | + | + | + | + | + | + |
| Sample 6 | + | + | + | + | + | + |
| Sample 7 | + | + | + | + | + | + |
| Sample 8 | - | - | + | + | + | + |
| Sample 9 | + | + | + | + | + | + |
| Sample 10 | + | + | + | + | + | + |
| Sample 11 | + | + | + | + | + | + |
| Sample 12 | + | + | + | + | + | + |
| Sample 13 | + | + | + | + | + | + |
| Sample 14 | + | + | + | + | + | + |
| Sample 15 | + | + | + | + | + | + |
| Sample 16 | + | + | + | + | + | + |
| Sample 17 | + | + | + | + | + | + |
| Sample 18 | + | + | + | + | + | + |
| Sample 19 | + | + | + | + | + | + |
| Sample 20 | + | + | + | + | + | + |
| Sample 21 | + | + | + | + | + | + |
| Sample 22 | + | + | + | + | + | + |
| Sample 23 | + | + | + | + | + | + |
| Sample 24 | + | + | + | + | + | + |

Table 3: cytopathic condition in 3: hAd5 neutralizing antibody experiment

| Ad5 | 1:4 | 1:8 | 1:16 | 1:32 | 1:64 | 1:128 |
|---|---|---|---|---|---|---|
| Positive sample | - | - | - | - | - | - |
| Inactive virus | + | - | - | - | - | - |
| Blank cell | - | - | - | - | - | - |
| Sample 1 | + | + | + | + | + | + |
| Sample 2 | - | - | - | - | - | - |
| Sample 3 | + | + | + | + | + | + |
| Sample 4 | - | - | - | - | - | + |

(continued)

| Ad5 | 1:4 | 1:8 | 1:16 | 1:32 | 1:64 | 1:128 |
|---|---|---|---|---|---|---|
| Sample 5 | + | + | + | + | + | + |
| Sample 6 | + | + | + | + | + | + |
| Sample 7 | - | - | - | - | - | - |
| Sample 8 | - | - | - | - | - | - |
| Sample 9 | - | - | - | - | - | + |
| Sample 10 | - | - | - | - | - | + |
| Sample 11 | - | - | - | - | - | - |
| Sample 12 | - | - | - | - | - | - |
| Sample 13 | - | - | - | - | - | + |
| Sample 14 | - | - | - | - | - | - |
| Sample 15 | - | - | - | - | - | - |
| Sample 16 | + | + | + | + | + | + |
| Sample 17 | + | + | + | + | + | + |
| Sample 18 | - | - | - | - | - | - |
| Sample 19 | - | - | + | + | + | + |
| Sample 20 | + | + | + | + | + | + |
| Sample 21 | - | - | - | - | - | - |
| Sample 22 | + | + | + | + | + | + |
| Sample 23 | + | + | + | + | + | + |
| Sample 24 | - | - | - | - | - | - |
| result | Among the 24 subjects, 15 subjects had neutralizing antibodies with the titer of 1: 8 to 1: 128 or above, accounting for 62.5% | | | | | |

The results showed that, as shown in Table 2 and Fig. 26, the use of the novel chimpanzee adenovirus sAd-AY01 produced no neutralizing antibody in 24 serum samples of the population, indicating that there was no antibody against the novel chimpanzee adenovirus in the population. As shown in Table 3 and Fig. 27, using human adenovirus type 5, 15 of 24 serum samples had neutralizing antibody with the neutralization titer of 1: 8-1: 128 or above, accounting for 62.5% of the total number. This indicates that if the novel chimpanzee adenovirus sAd-AY01 is used as a vaccine vector, pre-existing antibodies in the population can be avoided and a better immune effect can be achieved.

**Example 13 immunological evaluation of recombinant novel chimpanzee adenovirus psAd-S vaccine in a mouse model**

13.1 Detection of vaccine humoral immune response

Twenty SPF-grade mice (6-8 weeks of age) were randomly divided into three groups, five for each group. Mice were immunized with psAd-S vaccine according to the groupings shown in Table 4. The injection method was as follows: intramuscular injection was performed on the medial aspect of the posterior thigh. Injection dose: 100ul.

Table 4: Groups of Vaccinated Mice

| grou p | Vector vaccine | dosage | Immune mode | Number of mice |
|---|---|---|---|---|
| Hig h dose | psAd-S | 1*10^8 FFU | intramuscular injection | five |
| low dose | psAd-S | 1*10^7 FFU | intramuscular injection | five |
| cont rol | psAd△E1△ E3 | 1*10^7 FFU | intramuscular injection | five |

Blood was collected 14 days after immunization and serum was separated from SARS-CoV-2(2019-nCoV)Spike S1 antibody titer detection kit (mouse), Art.No. KIT007, for detecting anti-SARS-CoV-2 (2019-nCOV) Spikes1 antibody level in mouse serum samples.

The detection principle was as follows: an indirect ELSA method was used to coat novel coronavirus S1 protein on a solid phase carrier, and a sample to be detected was added for incubation. The anti-novel coronavirus antibody in the sample was combined with the protein on the solid phase carrier, and after plate washing, the anti-mouse IgG antibody labeled with horseradish peroxidase (HRP) was added to form an antigen-antibody-enzyme labeled antibody complex. After coloration with the substrate TMB, TMB is converted to blue under the catalysis of peroxidase and finally yellow under the action of acid, and the absorbance (OD value) at 450m is read, and the OD value at 450m is positively correlated with the anti-novel coronavirus antibody content in the sample.

Calculate an enzyme label strip required for detect a sample, taking out that enzyme label strip from the aluminum foil bag, putting the rest of the enzyme label strip back into the aluminum foil bag and seal the bag mouth, preserving at low temperature, washing the plate with 1 * washing buffer solution and soaking 1min at 300 mu/hole, patting the enzyme label plate for the next cleaning, adding the sample to be detected into the plate for five times of washing, diluting serum with 1 * dilution buffer solution at 1:2000, incubating at 100/ hole for 2 hours at room temperature, discarding the liquid in the hole, patting the enzyme label plate for drying, washing the plate with 1 * washing buffer solution and soaking at 300uL/ hole for 1min, and patting for drying The anti-mouse IgG antibody, -HRP1:100, was diluted with 1x dilution buffer, and 100ul/ well was added to the ELISA plate, mixed, and incubated for 1 hour at room temperature. The liquid in the wells was discarded, and the ELISA plate was pat dry. The plate was washed with $1\times$ washing buffer solution, and 1min was immersed in 300$\mu$l/ well. The ELISA plate was pat dry for the next washing, and the plate was washed five times in total. The pre-prepared substrate solution was added into the ELISA plate at 200$\mu$l/ well, mixed evenly, and incubated for 20 minutes at room temperature in the dark. Add 501 well stop solution to the ELISA plate and gently shake the plate until uniform color development is achieved. Read the light absorption value for OD450mm.

The test results are shown in Fig. 28. Two weeks after the intramuscular injection of psAd-S vaccine, the mice were able to produce high concentrations of IgG antibodies against spike protein. The serum of mice injected with 1 * 108 FFU dose was significantly different from that of the negative control group, and the serum of mice injected with 1 * 107 FFU dose was also significantly different from that of the control, and the humoral immune response induced by the high dose group was higher, (ns ,P$\geq$0 .05; * ,P<0 .05; ** ,P<0.01; ***,P<0.001; ****,P<0.0001)

13.2 Detection of cellular immune response

Ten SPF-grade mice (6-8 weeks of age) were randomly divided into two groups, five for each group. Mice were immunized with psAd-S vaccine according to the groupings shown in Table 5. The injection method was as follows: intramuscular injection was performed on the medial aspect of the posterior thigh. Injection dose: 100ul.

Table 5: Groups of Vaccinated Mice

| group | Vector vaccine | dosage | Immune mode | Number of mice |
|---|---|---|---|---|
| experimenta l group | psAd-S | 1*10^7 FFU | intramuscular injection | five |
| Control group | psAd$\triangle$E1$\triangle$ E3 | 1*10^7 FFU | intramuscular injection | five |

The mice were sacrificed 14 days after immunization, and the splenic lymphocytes were isolated. BHK21 cells transfected with the shuttle plasmid pSsAdE1-WPRE-S were stimulated and cultured for 6 hours, and cytokine secretion was blocked by adding a protein secretion blocker. After 6 hours, Fc receptors were blocked, dead cells and cell surface molecular markers were stained, and intracellular cytokines were stained after the cells were fixed and perforated. Cell surface markers included CD4 and CD8, and intracellular cytokines included IFN$\gamma$, IL2 and TNF. The expression levels of IFN$\gamma$, IL2 and TNF in CD4+T cells and CD8+T cells stimulated by the target protein were analyzed by flow cytometry (CyExpert).

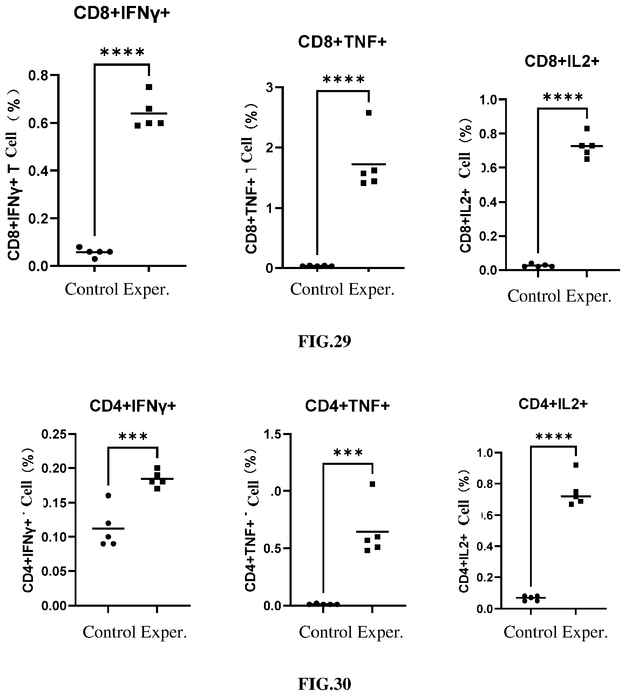

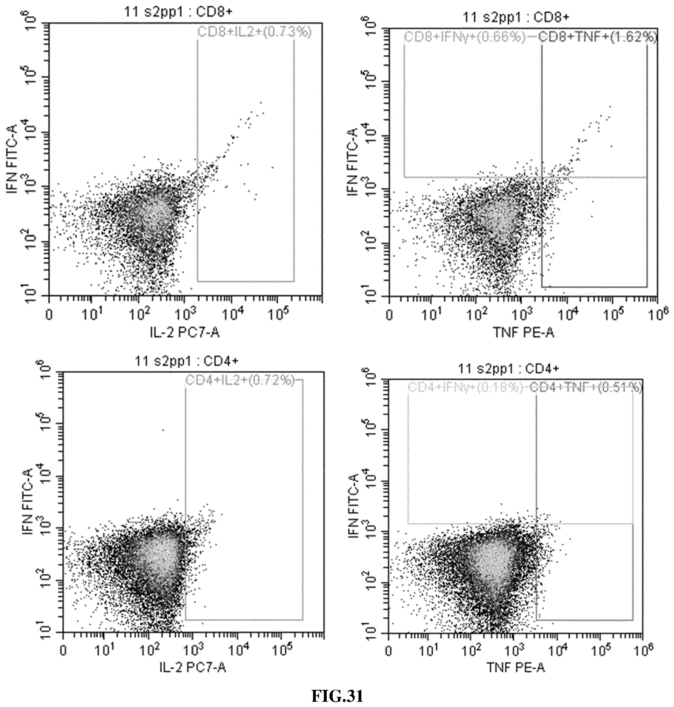

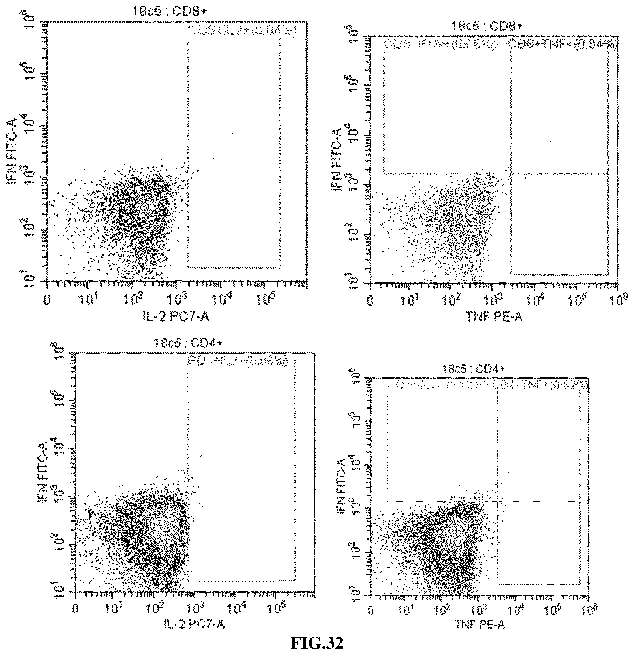

psAd-S-induced CD8+T-cell and CD4+T-cell immune responses are shown in Figs. 29 and 30, and representative results are shown in Figs. 31 and 32. Fig. 31 is a representative diagram of cellular immune response after intramuscular injection of psAd-S, and Fig. 32 is a representative diagram of blank control immune response. The results showed that, 14 days after immunization, after the spleen cells were stimulated by Spike protein, the levels of IFN$\gamma$, TNF$\alpha$ and IL2 expressed in CD8+T cells were significantly higher than those in the psAd$\triangle$E1$\triangle$E3 vehicle control group (Control)(P<0 .05). After stimulation, the expression levels of IFN$\gamma$, TNF$\alpha$ and IL2 in CD4+T cells were also significantly higher than those in the PSAD $\triangle$ E1 $\triangle$ E3 vehicle control group (Control)(P<0 .05).

13.3 SARS-CoV-2 Pseudovirus Simulated Neutralization Antibody Experiment

SARS-CoV-2 pseudovirus neutralization test kit is purchased from Nanjing Kingsri Biotechnology Co., Ltd. on the principle that the envelope glycoprotein in the lentivirus vector is replaced by Covid-19 S protein to form a pseudovirus simulating Covid-19 infection. Pseudoviruses infect target cells through surface S proteins and express reporter luciferase genes. Neutralizing agents (such as antibodies) can block the binding of S protein and ACE2, thereby preventing pseudovirus infection of the host cells. By detecting the expression level of the reporter gene luciferase, the degree to which the virus is blocked can be deduced for screening or verification of the neutralizing agent.

The experimental steps were as follows: The mouse serum, positive control and negative control collected in the cell immune response test were diluted and mixed with the diluted HRP-RBD in a 1:1 manner and incubated at 37 C for 30 minutes. Incubate 100 U of the mixture in the corresponding well plate for 15 minutes at 37 C; Wash the well plates 4 times with 260ul of 1X Wash buffer; Then 100ul TMB buffer was added and incubated for 15 min at 20-25 C in the dark. Adding 50ul of termination buffer; OD450 reads the absorbance. The result are shown in table 6:

Table 6: Simulated Neutralization Antibody Test of SARS-CoV-2 Pseudovirus

| serum | OD450 | suppre ssion ratio | Dilution multiple | result |
|---|---|---|---|---|
| 10 8 1 | 0.2672 | 81.9% | 256 | positive |
| 10 8 2 | 0.5680 | 61.6% | 256 | positive |
| 10 8 3 | 0.6779 | 54.1% | 256 | positive |
| 10 8 4 | 0.4522 | 69.4% | 256 | positive |
| 10 8 5 | 0.6728 | 54.5% | 256 | positive |
| 10 7 1 | 0.9243 | 37.5% | 256 | positive |
| 10 7 2 | 1.0025 | 32.2% | 256 | positive |
| 10 7 3 | 0.9389 | 36.5% | 256 | positive |
| 10 7 4 | 1.0420 | 29.5% | 256 | positive |
| 10 7 5 | 0.9300 | 37.1% | 256 | positive |
| positive control | 0.0816 | \ | 10 | \ |
| negative control | 1.4780 | \ | 10 | \ |

Among them: quality control: positive control < 0.3, negative control > 1.0; Inhibition rate = (1 - sample OD value/negative control OD value) * 100%; Judgement: Positive inhibition rate ≥20% and negative inhibition rate < 20%.

In summary, after mice were injected with the total dose of 10^8 FFU vaccine (diluted with PBS buffer) and 10^7 FFU (diluted with PBS buffer), they could induce strong humoral and cellular immune responses, which were significantly different from those in the control group. At the same time, the collected serum can play a blocking role in the simulated neutralization antibody experiment. The results showed that our psad-S vaccine could protect Covid-19, which had guiding significance for the development of vaccine in COVID-19.

Although the present invention has been disclosed as above, the present invention is not limited thereto. Various changes and modifications may be made by those skilled in the art without departing from the spirit and scope of the present invention, and the scope of the present invention should be determined by the scope of the appended claims.

**Claims**

1. A chimpanzee adenovirus is **characterized in that** the virus comprises a hypervariable region HVR, wherein, the hypervariable region HVR comprises any one or more nucleotide sequences represented by sequence numbers Seq ID NO. 9, Seq ID NO. 10, Seq ID NO. 11, Seq ID NO. 12, Seq ID NO. 13, or Seq ID NO. 14, or a nucleotide sequence having more than 50% homology with the above sequences.

2. The chimpanzee adenovirus according to claim 1, **characterized in that** the adenovirus comprises a region of the hypervariable region HVR7-9, and the hypervariable region HVR7-9 has a nucleotide sequence as shown in the sequence table Seq ID NO. 15.

3. The chimpanzee adenovirus according to any one of claims 1-2, **characterized in that** the hypervariable region HVR has a nucleotide sequence as shown in the sequence table Seq ID NO. 7 or a nucleotide sequence having more than 50% homology with the sequence.

4. The chimpanzee adenovirus according to any one of claims 1-3, **characterized in that** the virus further comprises a conserved region.

5. The invention relates to a chimpanzee adenovirus, which is **characterized in that** the virus has a preservation number of CCTCC NO: V202120 and a strain of sAd-AY01.

6. The chimpanzee adenovirus according to claim 5, **characterized in that** the chimpanzee adenovirus has a nucleotide sequence as shown in the sequence table Seq ID NO.1.

7. A replication defective chimpanzee adenovirus vector is **characterized in that** the chimpanzee adenovirus according to any one of claims 1 to 6 has a gene deletion in a conserved region.

8. The replication defective chimpanzee adenovirus vector according to Claim 7, **characterized in that** the deletion gene comprises any one or more of E1, E2A, E2B, E3 and E4.

9. The replication-deficient chimpanzee adenovirus vector according to claim 8, **characterized in that** the E1 and E3 genes are deleted and the nucleotide sequence is shown in the sequence table Seq ID NO. 4.

10. The invention relates to a method for constructing a replication-defective chimpanzee adenovirus vector, which is **characterized in that** the method comprises the following steps:

(1) Constructing shuttle plasmid psAd-shuttle, carrying out homologous recombination with chimpanzee adenovirus gene according to any one of claims 1 to 6 after linearization, and preparing ring plasmid psAd;
(2) Knock out the E1 gene of psAd to obtain the chimpanzee adenovirus vector psAd E1 with deletion of E1 gene;
(3) The chimpanzee adenovirus vector psAd E1 E3 was obtained by knocking out the E3 gene of psAd E1.

11. The construction method according to claim 10, **characterized in that** the shuttle plasmid psAd-shuttle in step 1) has a nucleotide sequence as shown in the sequence table Seq ID NO. 3 and the cyclic plasmid psAd has a nucleotide sequence as shown in the sequence table Seq ID NO. 2.

12. The construction method according to claim 11, **characterized in that** step 2) digests the E1 gene of psAd by CRISPR/Cas9, inserts an HRPT sequence containing a PsiI digestion site, purifies and ligates to obtain psAd E1, and the HRPT sequence has a nucleotide sequence as shown in the sequence table Seq ID NO. 5.

13. The construction method according to claim 12 is **characterized in that** step 3) the product obtained by digesting psAd E1 with CRISPR/Cas9 is seamlessly cloned with the fusion fragment pVIII-U-exon to obtain psAd E1E3; the fusion fragment pVIII-U-exon has a nucleotide sequence as shown in the sequence table Seq ID NO. 6.

14. A vaccine comprising a target gene in a gene deletion region of a replication-defective chimpanzee adenovirus vector according to any one of claims 7 to 9.

15. The vaccine according to claim 14, **characterized in that** the gene deletion region comprises any one or more of E1, E2A, E2B, E3 and E4.

16. The vaccine according to claim 15, **characterized in that** the target gene is a virus, a bacterium, a tumor gene or a gene fragment.

17. The vaccine according to claim 16, **characterized in that** the target gene is Novel Coronavirus S gene.

18. The vaccine according to claim 17, **characterized in that** the target gene is located in the E1 region of the replication-defective chimpanzee adenovirus vector, and the replication-defective chimpanzee adenovirus vector has a nucleotide sequence as shown in the sequence table Seq ID NO. 4.

19. The invention relates to a titer determination method of chimpanzee adenovirus, **characterized in that** the titer of chimpanzee adenovirus is detected by detecting the change of HVR antigen epitope in the hypermutant region through a specific antibody prepared from a hexon hypermutant region HVR gene fragment, wherein the HVR gene has a nucleotide sequence as shown in the sequence table Seq ID NO. 7.

20. The titer determination method according to claim 19, **characterized in that** the chimpanzee adenovirus is the chimpanzee virus according to any one of claims 1 to 6, or the replication-defective chimpanzee adenovirus vector according to any one of claims 7 to 9, or the vaccine according to any one of claims 14 to 18.

**FIG.1**

**FIG.2**

**FIG.3**

Percent Identity

| | 1 | 2 | 3 | 4 | 5 | 6 | 7 | 8 | 9 | 10 | 11 | 12 | 13 | | |
|---|---|---|---|---|---|---|---|---|---|---|---|---|---|---|---|
| 1 | ■ | 78.1 | 76.5 | 77.9 | 100.0 | 39.4 | 74.1 | 73.9 | 73.9 | 73.9 | 72.6 | 70.4 | 61.6 | 1 | Simian adenovirus 21.seq |
| 2 | 26.1 | ■ | 89.1 | 98.5 | 78.1 | 42.0 | 77.1 | 76.8 | 78.7 | 78.7 | 73.9 | 68.9 | 67.5 | 2 | Simian adenovirus 22.seq |
| 3 | 28.5 | 11.8 | ■ | 89.0 | 76.5 | 40.9 | 77.1 | 76.1 | 77.8 | 77.8 | 74.2 | 69.4 | 61.2 | 3 | Simian adenovirus 23.seq |
| 4 | 26.4 | 1.6 | 11.9 | ■ | 77.9 | 42.1 | 77.3 | 76.8 | 78.7 | 78.7 | 73.9 | 68.9 | 68.2 | 4 | Simian adenovirus 24.seq |
| 5 | 0.0 | 26.1 | 28.5 | 26.4 | ■ | 39.4 | 74.1 | 73.9 | 73.9 | 73.9 | 72.6 | 70.4 | 61.6 | 5 | Simian adenovirus 25.seq |
| 6 | 124.5 | 111.9 | 116.7 | 111.4 | 124.5 | ■ | 37.5 | 38.2 | 37.2 | 37.2 | 37.4 | 36.3 | 91.4 | 6 | Simian adenovirus 26.seq |
| 7 | 32.0 | 27.5 | 27.5 | 27.1 | 32.0 | 134.6 | ■ | 84.7 | 81.1 | 81.1 | 77.8 | 71.8 | 56.2 | 7 | Simian adenovirus 2.seq |
| 8 | 32.3 | 27.9 | 29.0 | 27.9 | 32.3 | 130.7 | 17.3 | ■ | 80.8 | 80.8 | 78.0 | 71.8 | 56.4 | 8 | Simian adenovirus 7.seq |
| 9 | 32.4 | 25.1 | 26.4 | 25.2 | 32.4 | 136.7 | 21.8 | 22.4 | ■ | 100.0 | 75.9 | 72.0 | 49.8 | 9 | Simian adenovirus 8.seq |
| 10 | 32.4 | 25.1 | 26.4 | 25.2 | 32.4 | 136.7 | 21.8 | 22.4 | 0.0 | ■ | 75.9 | 72.0 | 49.8 | 10 | Simian adenovirus 18.seq |
| 11 | 34.4 | 32.3 | 31.9 | 32.4 | 34.4 | 135.9 | 26.6 | 26.2 | 29.3 | 29.3 | ■ | 74.1 | 54.2 | 11 | Simian adenovirus 19.seq |
| 12 | 38.0 | 40.7 | 39.7 | 40.6 | 38.0 | 142.7 | 35.6 | 35.7 | 35.3 | 35.3 | 32.1 | ■ | 54.1 | 12 | Simian adenovirus 20.seq |
| 13 | 54.3 | 42.9 | 56.1 | 41.8 | 54.3 | 9.2 | 66.2 | 65.5 | 85.2 | 85.2 | 71.6 | 71.3 | ■ | 13 | sAd-AY01.seq |
| | 1 | 2 | 3 | 4 | 5 | 6 | 7 | 8 | 9 | 10 | 11 | 12 | 13 | | |

Divergence

**FIG.4**

**FIG.5-1**

28

HAdV- 14.seq
HAdV- 34.seq
HAdV- 35.seq
HAdV- 21.seq
Simian adenovirus 21.seq
HAdV- 50.seq
Simian adenovirus 25.seq
HAdV- 3.seq
HAdV- 7.seq
HAdV- 46.seq
HAdV- 48.seq
HAdV- 49.seq
HAdV- 26.seq
HAdV- 37.seq
HAdV- 9.seq
HAdV- 4.seq
HAdV- 16.seq
Pan troglodytes adenovirus clone 17.3.s
Pan troglodytes adenovirus clone 17.2.s
Pan troglodytes adenovirus  CU- 2013.seq
sAd- AY01.seq
Simian adenovirus 26.seq
Simian adenovirus 22.seq
Simian adenovirus 24.seq
Simian adenovirus 23.seq
HAdV- 2.seq
HAdV- 5.seq
HAdV- 1.seq
Simian adenovirus 8.seq
Simian adenovirus 18.seq
Simian adenovirus 2.seq
Simian adenovirus 7.seq
Simian adenovirus 19.seq
HAdV- 40.seq
HAdV- 54.seq
Simian adenovirus 20.seq
HAdV- 12.seq

58.7

50   40   30   20   10   0

Nucleotide Substitutions (x100)

**FIG.5-2**

1. psAd-shuttle: EcoRI/BamHI

FIG.6

left arm

right arm

psAd-shuttle
(6451 bp)

BGHployA

pACYC origin

EGFP

RSV promoter

kanamycin

FIG.7

1. psAd: BamHI
2. psAd: XhoI

**FIG.8**

pACYC origin  kanamycin
RSV promoter
EGFP
BGHployA

psAd
（40224 bp）

sAd-AY01

**FIG.9**

**FIG.10**

**FIG.11**

1. psAd:BamHI
2. psAd:XhoI
3. psAd△E1:BamHI
4. psAd△E1:XhoI

15000pb

5000bp
2500bp

1000bp

250bp

**FIG.12**

15000bp

5000bp

2500 bp

1000bp

250bp

**FIG.13**

15000pb

5000bp
2500bp

1000bp

250bp

**FIG.14**

15000pb

5000bp

2500bp

1000bp

250bp

**FIG.15**

1.  psAd△E1△E3:BamHI
2.  psAd△E1△E3:XhoI

10000—
5000—
2500—
2000—
1500—
1000—
500—

FIG.16

pACYC origin
kanamycin
RSV promoter
EGFP
BGHployA
HRPT

fiber

100k

psAd△E1△E3
(32146bp)

DNA binding protein

hexon
pVI

52/55 kDa

pV penton pIIIa

FIG.17

**FIG.18**

1: pSsAdE1:XhoI

**FIG.19**

**FIG.20**

1: pSsAdE1-WPRE-S:EcoRV/HindIII

**FIG.21**

1: psAdE1-S:BamHI
2: psAdE1-S:HindIII

**FIG.22**

**FIG.23**

**FIG.24**

**FIG.25**

The titer of the antibody to the chimpanzee adenovirus sAd-AY01 in the serum of human

**FIG.26**

The titer of the antibody to the hAd5 in the serum of human

**FIG.27**

# IgG

**FIG.28**

**CD8+IFNγ+**

****

**CD8+TNF+**

****

**CD8+IL2+**

****

**FIG.29**

**CD4+IFNγ+**

***

**CD4+TNF+**

***

**CD4+IL2+**

****

**FIG.30**

**FIG.31**

FIG.32

## INTERNATIONAL SEARCH REPORT

| International application No. |
| --- |
| **PCT/CN2021/104641** |

**A.     CLASSIFICATION OF SUBJECT MATTER**

C12N 15/34(2006.01)i;  C12N 15/861(2006.01)i;  A61K 48/00(2006.01)i;  A61P 35/00(2006.01)i

According to International Patent Classification (IPC) or to both national classification and IPC

**B.      FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

VEN, TWTXT, USTXT, WOTXT, SpringerLink, ISI Web of Knowledge, Elsevier Science Direct, Wiley InterScience, China Patents Biological Sequence Search System, Embase, NCBI, EBI, Unipro, Nature, Science, PUBMED, CNKI, 万方, Seq2, 7-15, 黑猩猩腺病毒, 腺病毒, 载体, 疫苗, 载运体, s蛋白, 新冠病毒, 复制缺陷性, HVR, E1, E3, pACYC, chimpanzee adenoviral vector, vaccine, replication defective, deletion.

**C.      DOCUMENTS CONSIDERED TO BE RELEVANT**

| Category* | Citation of document, with indication, where appropriate, of the relevant passages | Relevant to claim No. |
| --- | --- | --- |
| X | CN 107723298 A (MSD ITALY) 23 February 2018 (2018-02-23) description pages 2-6, 12-13, 20, SEQ ID NO:2, 43 | 1-20 |
| X | CN 105112428 A (OKAIROS AG) 02 December 2015 (2015-12-02) claims 1-19, SEQ ID NO:38-41 | 1-20 |
| X | WO 2019076882 A1 (GLAXOSMITHKLINE BIOLOGICALS SA) 25 April 2019 (2019-04-25) claims 1-27, SEQ ID NO:2 | 1-20 |
| A | CN 111108192 A (NOUSCOM AG) 05 May 2020 (2020-05-05) entire document | 1-20 |
| A | CN 102844329 A (MOGAM BIOTECH RES INST.) 26 December 2012 (2012-12-26) entire document | 1-20 |
| A | CN 111363858 A (NANJING LIMING BIO-PRODUCTS CO., LTD.) 03 July 2020 (2020-07-03) entire document | 14-18 |

☑ Further documents are listed in the continuation of Box C.     ☑ See patent family annex.

| | |
| --- | --- |
| *      Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **24 September 2021** | **11 October 2021** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/104641** |

**C.    DOCUMENTS CONSIDERED TO BE RELEVANT**

| Category* | Citation of document, with indication, where appropriate, of the relevant passages | Relevant to claim No. |
| --- | --- | --- |
| A | KIM, Y.C. et al. "COVID-19 Vaccines: Breaking Record Times To First-in-human Trials" *NPJ VACCINES*, 30 April 2020 (2020-04-30), article no.34, 1-3 | 1-18 |

Form PCT/ISA/210 (second sheet) (January 2015)

**INTERNATIONAL SEARCH REPORT**

International application No.

**PCT/CN2021/104641**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

46

**INTERNATIONAL SEARCH REPORT**
Information on patent family members

| International application No. |
| --- |
| **PCT/CN2021/104641** |

| Patent document cited in search report | | | Publication date (day/month/year) | Patent family member(s) | | | Publication date (day/month/year) |
| --- | --- | --- | --- | --- | --- | --- | --- |
| CN | 107723298 | A | 23 February 2018 | WO | 2005071093 | A2 | 04 August 2005 |
| | | | | PT | 2163260 | T | 08 June 2017 |
| | | | | EP | 3269390 | B1 | 17 February 2021 |
| | | | | ES | 2337374 | T3 | 23 April 2010 |
| | | | | SI | EP2163260 | T1 | 31 July 2017 |
| | | | | CN | 102719478 | A | 10 October 2012 |
| | | | | EP | 2163260 | A3 | 23 June 2010 |
| | | | | JP | 5427874 | B2 | 26 February 2014 |
| | | | | SI | 1711518 | T1 | 30 April 2010 |
| | | | | WO | 2005071093 | A3 | 30 March 2006 |
| | | | | CA | 2880061 | A1 | 04 August 2005 |
| | | | | CA | 2880060 | A1 | 04 August 2005 |
| | | | | DK | 1711518 | T3 | 06 April 2010 |
| | | | | EP | 1711518 | B1 | 18 November 2009 |
| | | | | AU | 2005206292 | B2 | 29 September 2011 |
| | | | | EP | 2163260 | A2 | 17 March 2010 |
| | | | | CA | 2880060 | C | 13 March 2018 |
| | | | | JP | 5753377 | B2 | 22 July 2015 |
| | | | | CY | 1109841 | T1 | 10 September 2014 |
| | | | | CA | 2993042 | A1 | 04 August 2005 |
| | | | | AU | 2011247884 | B2 | 20 November 2014 |
| | | | | US | 8216834 | B2 | 10 July 2012 |
| | | | | AT | 449105 | T | 15 December 2009 |
| | | | | LT | 2163260 | T | 26 June 2017 |
| | | | | JP | 2007518414 | A | 12 July 2007 |
| | | | | JP | 2012110326 | A | 14 June 2012 |
| | | | | EP | 3269390 | A3 | 18 April 2018 |
| | | | | CA | 2553541 | A1 | 04 August 2005 |
| | | | | US | 8673319 | B2 | 18 March 2014 |
| | | | | JP | 4814800 | B2 | 16 November 2011 |
| | | | | PL | 1711518 | T3 | 30 June 2010 |
| | | | | US | 2012328651 | A1 | 27 December 2012 |
| | | | | EP | 3269390 | A2 | 17 January 2018 |
| | | | | AU | 2005206292 | A1 | 04 August 2005 |
| | | | | HU | E033576 | T2 | 28 December 2017 |
| | | | | SI | EP1711518 | T1 | 30 April 2010 |
| | | | | SI | 2163260 | T1 | 31 July 2017 |
| | | | | EP | 1711518 | A2 | 18 October 2006 |
| | | | | CA | 2553541 | C | 21 April 2015 |
| | | | | EP | 2163260 | B1 | 15 March 2017 |
| | | | | CA | 2880061 | C | 13 March 2018 |
| | | | | DK | 2163260 | T3 | 19 June 2017 |
| | | | | PT | 1711518 | E | 26 February 2010 |
| | | | | JP | 2014158467 | A | 04 September 2014 |
| | | | | CN | 101014613 | A | 08 August 2007 |
| | | | | JP | 2011120588 | A | 23 June 2011 |
| | | | | CA | 3028774 | A1 | 04 August 2005 |
| | | | | CN | 101014613 | B | 05 September 2012 |
| | | | | US | 2011217332 | A1 | 08 September 2011 |
| CN | 105112428 | A | 02 December 2015 | WO | 2010085984 | A1 | 05 August 2010 |

Form PCT/ISA/210 (patent family annex) (January 2015)

<table>
<tr><td colspan="2" rowspan="2">INTERNATIONAL SEARCH REPORT<br>Information on patent family members</td><td colspan="2">International application No.</td></tr>
<tr><td colspan="2">**PCT/CN2021/104641**</td></tr>
</table>

| Patent document<br>cited in search report | | | Publication date<br>(day/month/year) | Patent family member(s) | | | Publication date<br>(day/month/year) |
|---|---|---|---|---|---|---|---|
| | | | | CN | 105112428 | B | 20 April 2021 |
| | | | | NZ | 603348 | A | 30 May 2014 |
| WO | 2019076882 | A1 | 25 April 2019 | CA | 3079048 | A1 | 25 April 2019 |
| | | | | BR | 112020007008 | A2 | 17 November 2020 |
| | | | | EP | 3697919 | A1 | 26 August 2020 |
| | | | | JP | 2020537526 | A | 24 December 2020 |
| | | | | CN | 111527213 | A | 11 August 2020 |
| CN | 111108192 | A | 05 May 2020 | EP | 3649237 | A1 | 13 May 2020 |
| | | | | WO | 2019008111 | A1 | 10 January 2019 |
| | | | | BR | 112020000145 | A2 | 14 July 2020 |
| | | | | US | 2021130848 | A1 | 06 May 2021 |
| | | | | CA | 3066962 | A1 | 10 January 2019 |
| | | | | KR | 20200024296 | A | 06 March 2020 |
| | | | | IL | 271835 | D0 | 27 February 2020 |
| | | | | SG | 11201913178 W | A | 30 January 2020 |
| | | | | JP | 2020530761 | A | 29 October 2020 |
| | | | | AU | 2018295421 | A1 | 02 January 2020 |
| CN | 102844329 | A | 26 December 2012 | CN | 102844329 | B | 20 January 2016 |
| | | | | US | 10066215 | B2 | 04 September 2018 |
| | | | | EP | 2558481 | A4 | 02 October 2013 |
| | | | | JP | 5785607 | B2 | 30 September 2015 |
| | | | | WO | 2011129468 | A1 | 20 October 2011 |
| | | | | US | 2015111282 | A1 | 23 April 2015 |
| | | | | KR | 20120137508 | A | 21 December 2012 |
| | | | | EP | 2558481 | B1 | 20 December 2017 |
| | | | | US | 9493745 | B2 | 15 November 2016 |
| | | | | WO | 2011129468 | A9 | 15 November 2012 |
| | | | | US | 2017051256 | A1 | 23 February 2017 |
| | | | | US | 2013058897 | A1 | 07 March 2013 |
| | | | | KR | 101458367 | B1 | 05 November 2014 |
| | | | | JP | 2013523173 | A | 17 June 2013 |
| | | | | EP | 2558481 | A1 | 20 February 2013 |
| CN | 111363858 | A | 03 July 2020 | CN | 111363858 | B | 29 September 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2020106427453

- CN 202010945335

**Non-patent literature cited in the description**

- **HILLIS WD ; GOODMAN R.** *Serological classification of chimpanzee adenoviruses by hemagglutination and hemagglutination inhibition,* 1969
- **CASAS A ; AVELLON M ; MOSQUERA O.** Molecular typing of human adenoviruses by PCR and sequencing of a partial region of the hexon gene. Lu X, Erdman DD. Molecular typing of human adenoviruses by PCR and sequencing of a partial region of the hexon gene. *Arch Viro 1,* 2006, vol. 151, 1587-1602

- **RIKA MO ; YASUSHI S ; TSUNETADA K.** Quantitative Detection and Rapid Identification of Human Adenoviruses. *J Clin Microbio1.,* 2007, vol. 4 5 (9 5 8), 967